(19) **Europäisches Patentamt / European Patent Office / Office européen des brevets**

(11) **EP 4 056 233 A1**

(12) **EUROPEAN PATENT APPLICATION**
published in accordance with Art. 153(4) EPC

(43) Date of publication:
**14.09.2022 Bulletin 2022/37**

(21) Application number: **20861450.3**

(22) Date of filing: **04.09.2020**

(51) International Patent Classification (IPC):
**A61P 43/00** (2006.01)          **C07K 1/02** (2006.01)
**C07K 7/04** (2006.01)          **A61K 47/42** (2017.01)
**C12N 15/12** (2006.01)          **A61K 38/06** (2006.01)
**A61K 38/07** (2006.01)          **A61K 38/08** (2019.01)
**A61K 38/10** (2006.01)

(52) Cooperative Patent Classification (CPC):
**A61K 38/06; A61K 38/07; A61K 38/08;
A61K 38/10; A61K 47/42; A61P 43/00; C07K 1/02;
C07K 7/04**

(86) International application number:
**PCT/JP2020/033658**

(87) International publication number:
**WO 2021/045210 (11.03.2021 Gazette 2021/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.09.2019 JP 2019162119**

(71) Applicant: **AJINOMOTO CO., INC.
Chuo-ku
Tokyo 104-8315 (JP)**

(72) Inventors:
• **INOUE, Ippei
  Kawasaki-shi, Kanagawa 210-8681 (JP)**
• **ITO, Kenichiro
  Kawasaki-shi, Kanagawa 210-8681 (JP)**
• **TOKUYAMA, Mayumi
  Kawasaki-shi, Kanagawa 210-8681 (JP)**

(74) Representative: **Mewburn Ellis LLP
Aurora Building
Counterslip
Bristol BS1 6BX (GB)**

(54) **FERRITIN ENCLOSING PEPTIDE**

(57)    The present invention provides a means for transporting a peptide while avoiding destruction of cellular membrane, immunogenicity, and toxicity. More specifically, the present invention provides the following:
(1) a peptide-encapsulating ferritin including a peptide that is composed of 3 to 19 amino acid residues and satisfies the following requirement:
a) $-10.2 \leq X \leq 5.9$ and $445 \leq Y \leq 2,524$

wherein X represents a charge of the peptide at pH 9, and Y represents a chemical formula weight of the peptide;
(2) an agent for intracellularly transporting a peptide, including the peptide-encapsulating ferritin described above; and
(3) a method for producing the peptide-encapsulating ferritin described above.

EP 4 056 233 A1

[SEQUENCE LISTING]

SEQUENCE LISTING

<110> Ajinomoto Co., Inc.

<120> Peptide-encapsulating ferritin

<130> AM-R021787

<150> JP2019-162119
<151> 2019-09-05

<160> 28

<170> PatentIn version 3.5

<210> 1
<211> 183
<212> PRT
<213> Homo sapiens

<400> 1

```
Met Thr Thr Ala Ser Thr Ser Gln Val Arg Gln Asn Tyr His Gln Asp
1               5                   10                  15


Ser Glu Ala Ala Ile Asn Arg Gln Ile Asn Leu Glu Leu Tyr Ala Ser
            20                  25                  30


Tyr Val Tyr Leu Ser Met Ser Tyr Tyr Phe Asp Arg Asp Asp Val Ala
        35                  40                  45


Leu Lys Asn Phe Ala Lys Tyr Phe Leu His Gln Ser His Glu Glu Arg
    50                  55                  60


Glu His Ala Glu Lys Leu Met Lys Leu Gln Asn Gln Arg Gly Gly Arg
65                  70                  75                  80


Ile Phe Leu Gln Asp Ile Lys Lys Pro Asp Cys Asp Asp Trp Glu Ser
                85                  90                  95


Gly Leu Asn Ala Met Glu Cys Ala Leu His Leu Glu Lys Asn Val Asn
            100                 105                 110


Gln Ser Leu Leu Glu Leu His Lys Leu Ala Thr Asp Lys Asn Asp Pro
        115                 120                 125


His Leu Cys Asp Phe Ile Glu Thr His Tyr Leu Asn Glu Gln Val Lys
    130                 135                 140


Ala Ile Lys Glu Leu Gly Asp His Val Thr Asn Leu Arg Lys Met Gly
145                 150                 155                 160


Ala Pro Glu Ser Gly Leu Ala Glu Tyr Leu Phe Asp Lys His Thr Leu
```

165                    170                    175


Gly Asp Ser Asp Asn Glu Ser
              180


<210>   2
<211>   175
<212>   PRT
<213>   Homo sapiens

<400>   2

Met Ser Ser Gln Ile Arg Gln Asn Tyr Ser Thr Asp Val Glu Ala Ala
1               5                   10                  15


Val Asn Ser Leu Val Asn Leu Tyr Leu Gln Ala Ser Tyr Thr Tyr Leu
            20                  25                  30


Ser Leu Gly Phe Tyr Phe Asp Arg Asp Asp Val Ala Leu Glu Gly Val
            35                  40                  45


Ser His Phe Phe Arg Glu Leu Ala Glu Glu Lys Arg Glu Gly Tyr Glu
        50                  55                  60


Arg Leu Leu Lys Met Gln Asn Gln Arg Gly Gly Arg Ala Leu Phe Gln
65                  70                  75                  80


Asp Ile Lys Lys Pro Ala Glu Asp Glu Trp Gly Lys Thr Pro Asp Ala
                85                  90                  95


Met Lys Ala Ala Met Ala Leu Glu Lys Lys Leu Asn Gln Ala Leu Leu
            100                 105                 110


Asp Leu His Ala Leu Gly Ser Ala Arg Thr Asp Pro His Leu Cys Asp
            115                 120                 125


Phe Leu Glu Thr His Phe Leu Asp Glu Glu Val Lys Leu Ile Lys Lys
        130                 135                 140


Met Gly Asp His Leu Thr Asn Leu His Arg Leu Gly Gly Pro Glu Ala
145                 150                 155                 160


Gly Leu Gly Glu Tyr Leu Phe Glu Arg Leu Thr Leu Lys His Asp
                165                 170                 175


<210>   3
<211>   36
<212>   DNA
<213>   Artificial Sequence

```
<220>
<223>   GAAGGAGATATACATATGACGACCGCGTCCACCTCG


<400>   3
gaaggagata tacatatgac gaccgcgtcc acctcg                                36



<210>   4
<211>   36
<212>   DNA
<213>   Artificial Sequence


<220>
<223>   A primer for amplifying DNA encoding human ferritin heavy chain

<400>   4
ctcgaattcg gatccttagc tttcattatc actgtc                                36



<210>   5
<211>   33
<212>   DNA
<213>   Artificial Sequence


<220>
<223>   A primer for amplifying the vector pET20

<400>   5
tttcatatgt atatctcctt cttaaagtta aac                                   33



<210>   6
<211>   26
<212>   DNA
<213>   Artificial Sequence


<220>
<223>   A primer for amplifying the vector pET20

<400>   6
tttggatccg aattcgagct ccgtcg                                           26



<210>   7
<211>   36
<212>   DNA
<213>   Artificial Sequence


<220>
<223>   A primer for amplifying DNA encoding human ferritin light chain

<400>   7
gaaggagata tacatatgag ctcccagatt cgtcag                                36



<210>   8
<211>   36
<212>   DNA
<213>   Artificial Sequence


<220>
<223>   A primer for amplifying DNA encoding human ferritin light chain
```

```
<400>  8
ctcgaattcg gatccttagt cgtgcttgag agtgag                                          36
```

```
<210>  9
<211>  18
<212>  PRT
<213>  Artificial Sequence

<220>
<223>  An amino acid sequence of the peptide PKC-F which is to be tested
       for encapsulation with ferritin

<400>  9
```

Arg Phe Ala Arg Lys Gly Ala Leu Arg Gln Lys Asn Val His Glu Val
1               5                   10                  15


Lys Asn


```
<210>  10
<211>  11
<212>  PRT
<213>  Artificial Sequence

<220>
<223>  An amino acid sequence of the peptide SV40 which is to be tested
       for encapsulation with ferritin

<400>  10
```

Cys Gly Gly Pro Lys Lys Lys Arg Lys Val Gly
1               5                   10


```
<210>  11
<211>  20
<212>  PRT
<213>  Artificial Sequence

<220>
<223>  An amino acid sequence of the peptide Np which is to be tested
       for encapsulation with ferritin

<400>  11
```

Cys Gly Gly Lys Arg Pro Ala Ala Ile Lys Lys Ala Gly Gln Ala Lys
1               5                   10                  15


Lys Lys Lys Gly
                20


```
<210>  12
<211>  4
<212>  PRT
<213>  Artificial Sequence

<220>
```

<223> An amino acid sequence of the peptide EGFR which is to be tested for encapsulation with ferritin

<400> 12

His Thr Ser Asp
1


<210> 13
<211> 5
<212> PRT
<213> Artificial Sequence

<220>
<223> An amino acid sequence of the peptide cyloRGDfV which is to be tested for encapsulation with ferritin

<400> 13

Arg Gly Asp Phe Val
1                   5


<210> 14
<211> 7
<212> PRT
<213> Artificial Sequence

<220>
<223> An amino acid sequence of the peptide FGF3-FP16 which is to be tested for encapsulation with ferritin

<400> 14

Val Leu Trp Leu Lys Asn Arg
1                   5


<210> 15
<211> 8
<212> PRT
<213> Artificial Sequence

<220>
<223> An amino acid sequence of the peptide Hymenistatin I which is to be tested for encapsulation with ferritin

<400> 15

Pro Pro Tyr Val Pro Leu Ile Ile
1                   5


<210> 16
<211> 10
<212> PRT
<213> Artificial Sequence

<220>
<223> An amino acid sequence of the peptide Ex10 which is to be tested for encapsulation with ferritin

<400> 16

Glu Glu Glu Glu Glu Glu Glu Glu Glu Glu
1               5                   10


<210> 17
<211> 10
<212> PRT
<213> Artificial Sequence

<220>
<223> An amino acid sequence of the peptide Rx10 which is to be tested
for encapsulation with ferritin

<400> 17

Arg Arg Arg Arg Arg Arg Arg Arg Arg Arg
1               5                   10


<210> 18
<211> 13
<212> PRT
<213> Artificial Sequence

<220>
<223> An amino acid sequence of the peptide avb6 which is to be tested
for encapsulation with ferritin

<400> 18

Ser Pro Arg Gly Asp Leu Ala Val Leu Gly His Lys Tyr
1               5                   10


<210> 19
<211> 14
<212> PRT
<213> Artificial Sequence

<220>
<223> An amino acid sequence of the peptide NRP-1-2 which is to be
tested for encapsulation with ferritin

<400> 19

Gly Gly Lys Arg Pro Ala Arg Gly Gly Lys Arg Pro Ala Arg
1               5                   10


<210> 20
<211> 14
<212> PRT
<213> Artificial Sequence

<220>
<223> An amino acid sequence of the peptide Pakti-L1 which is to be
tested for encapsulation with ferritin.  A sulfur atom in side
chain of cysteine residue is covalently linked to an acetyl
group.

<400> 20

Tyr Ile Leu Val Arg Asn Arg Leu Leu Arg Val Asp Cys Gly
1               5                   10


<210>  21
<211>  16
<212>  PRT
<213>  Artificial Sequence

<220>
<223>  An amino acid sequence of the peptide NS which is to be tested
       for encapsulation with ferritin

<400>  21

Gln Met Ala Arg Ile Pro Lys Arg Leu Ala Arg His Gln Met Ala Arg
1               5                   10                  15


<210>  22
<211>  18
<212>  PRT
<213>  Artificial Sequence

<220>
<223>  An amino acid sequence of the peptide GRP78-3 which is to be
       tested for encapsulation with ferritin

<400>  22

Gly Ile Arg Leu Arg Gly Gly Ile Arg Leu Arg Gly Gly Ile Arg Leu
1               5                   10                  15


Arg Gly



<210>  23
<211>  20
<212>  PRT
<213>  Artificial Sequence

<220>
<223>  An amino acid sequence of the peptide FBP0032 which is to be
       tested for encapsulation with ferritin.  Two cysteine residues
       are covalently linked via disulfide bridge.  X at position 1
       indicates 4-pentynoic acid.


<220>
<221>  misc_feature
<222>  (1)..(1)
<223>  Xaa can be any naturally occurring amino acid

<400>  23

Xaa Gly Gln Lys Gly Cys Lys Lys Thr Pro Lys Leu Ala Ser Leu Ser
1               5                   10                  15


Cys Thr Gly Phe

20

```
<210>    24
<211>    20
<212>    PRT
<213>    Artificial Sequence

<220>
<223>    An amino acid sequence of the peptide FBP0033 which is to be
         tested for encapsulation with ferritin.  Two cysteine residues
         are covalently linked via disulfide bridge.  X at position 1
         indicates 4-pentynoic acid.


<220>
<221>    misc_feature
<222>    (1)..(1)
<223>    Xaa can be any naturally occurring amino acid

<400>    24

Xaa Gly Arg Pro Gly Cys Gly Pro Arg Lys Pro Arg Thr Pro Lys Lys
1               5                   10                  15


Cys Gly Ser His
            20


<210>    25
<211>    20
<212>    DNA
<213>    Artificial Sequence

<220>
<223>    A primer for amplifying DNA encoding transferrin receptor (TfR)

<400>    25
tggcagttca gaatgatgga                                                20


<210>    26
<211>    20
<212>    DNA
<213>    Artificial Sequence

<220>
<223>    A primer for amplifying DNA encoding transferrin receptor (TfR)

<400>    26
aggctgaacc gggtatatga                                                20


<210>    27
<211>    20
<212>    DNA
<213>    Artificial Sequence

<220>
<223>    A primer for amplifying DNA encoding 18S rRNA

<400>    27
```

```
tgagaaacgg ctaccacatc                                                    20


<210>  28
<211>  20
<212>  DNA
<213>  Artificial Sequence

<220>
<223>  A primer for amplifying DNA encoding 18S rRNA

<400>  28
ttacagggcc tcgaaagagt                                                    20
```

**Description**

TECHNICAL FIELD

The present invention relates to a peptide encapsulating ferritin and the like.

BACKGROUND ART

Peptides that targets intracellular proteins and nucleic acids and have physiological activities are expected to be applied to pharmaceutical products (Non Patent Literature 1). However, many of such peptides have problems in cellular membrane permeability, and various techniques of transporting them into cells are contemplated.

A feature of encapsulating an aimed peptide into nanoparticles and microparticles represented by liposomes and fusing liposomes to cellular membrane was reported as an approach to transporting the aimed peptide into cells (Patent Literature 1). A feature of fusing to cellular membrane permeable molecules and peptides and permeating them through cellular membrane or incorporating them into cells by using endosomes was also reported as an approach to transporting the aimed peptide into cells (Non Patent Literature 2).

Ferritin is a spherical protein that is ubiquitously present in organisms from animals and plants to microorganism and has an internal cavity formed by a plurality of monomers. In animals such as humans, it is known that two kinds of monomers of H and L chains are present as monomers constituting ferritin, and that ferritin is a multimer that includes 24 monomers (in many cases, a mixture of H chains and L chains) and has a cage form with an outer diameter of 12 nm and an internal cavity with an inner diameter of 7 nm. It is known that ferritin is deeply involved in homeostasis of an iron element in the living organisms or cells, and holds iron inside the internal cavity thereof for playing physiological functions such as transporting and storing iron. It is known that ferritin is incorporated into transferrin receptor-presenting cells (Non Patent Literature 3).

Ferritin is contemplated for its application as a DDS carrier (Patent Literature 2) that encapsulates a small organic compound into the internal cavity thereof, and utilized also in production of electronic devices.

PRIOR ART REFERENCES

PATENT LITERARURES

 Patent Literature 1: US Patent No. 4,515,736
 Patent Literature 2: Chinese Patent Application Laid-open NO. 106110333

Non Patent Literature

 Non Patent Literature 1: Y. Hayashi et al. ACS Chem. Biol. 2012; 7(3): 607-613
 Non Patent Literature 2: Z. Guo et al. Biomed Rep. 2016; 4(5): 528-534
 Non-Patent Literature 3: L. Li et al. Proc Natl Acad Sci USA. 2010; 107(8): 3505-10

SUMMARY OF INVENTION

PROBLEM TO BE SOLVED BY THE INVENTION

As described above, the use of liposomes or cell-permeable peptides was reported as an approach to transporting the aimed peptide into cells. However, in general, the liposomes and the cell-permeable peptides are positively charged in a neutral pH region, thereby causing a problem of destroying cellular membrane by interaction with the cellular membrane. Liposomes can exhibit immunogenicity, and metabolites of the liposomes can exhibit toxicity.

Accordingly, the present invention is aimed at providing a means for transporting a peptide while avoiding the abovementioned destruction of cellular membrane, immunogenicity, and toxicity.

MEANS FOR SOLVING PROBLEM

As a result of an extensive study, the inventors of the present application have found that a certain peptide can be encapsulated in ferritin, that such a peptide can be used for preparation of a peptide-encapsulating ferritin, and that such a peptide-encapsulating ferritin can be used for transporting a certain peptide into specific cells.

Ferritin is negatively charged in a neutral pH region and is incorporated into specific cells via a transferrin receptor. Therefore, with use of ferritin encapsulating the peptide therein, the abovementioned problems can be solved by transporting a certain peptide into the cells without destroying cellular membranes. Ferritin is a naturally occurring protein that is present naturally in mammals such as humans, thereby enabling it to solve the immunogenicity problem by coinciding a kind of ferritin (mammal species from which ferritin is derived) with mammal species to be administered with ferritin. From the viewpoint that ferritin is composed of naturally occurring amino acids and that metabolites of the naturally occurring amino acids are natural substances existing in vivo, the cytotoxicity problems resulting from the metabolites can also be solved since the metabolites of naturally occurring amino acids are unlikely to cause cytotoxicity.

As described above, the present inventors have succeeded in solving the above problems, and accomplished the present invention. That is, the present invention is as follows.

[1] A peptide-encapsulating ferritin comprising
a peptide that is composed of 3 to 19 amino acid residues and satisfies the following requirement:

a) $-10.2 \leq X \leq 5.9$ and $445 \leq Y \leq 2{,}524$

wherein X represents a charge of the peptide at pH 9, and Y represents a chemical formula weight of the peptide.

[2] The peptide-encapsulating ferritin according to [1], wherein the peptide is composed of 4 to 16 amino acid residues.

[3] The peptide-encapsulating ferritin according to [1] or [2], wherein the peptide satisfies the following requirement:

b) $-10.2 \leq X \leq 0.0$ and $445 \leq Y \leq 2{,}524$; or
c) $3.7 \leq X \leq 5.9$ and $445 \leq Y \leq 2{,}524$

wherein X and Y represent the same as those defined in [1] .

[4] An agent for intracellularly transporting a peptide, the agent comprising
a peptide-encapsulating ferritin, wherein the peptide is composed of 3 to 19 amino acid residues and satisfies the following requirement:

a) $-10.2 \leq X \leq 5.9$ and $445 \leq Y \leq 2{,}524$

wherein X represents a charge of the peptide at pH 9, and Y represents a chemical formula weight of the peptide.

[5] The agent according to [4], wherein the peptide is transported into a human cell.

[6] The agent according to [4] or [5], wherein the peptide is transported into a cancer cell.

[7] A method for producing a peptide-encapsulating ferritin, the method comprising:

1) standing a ferritin in a buffer with a pH of 3.0 or less to dissociate the ferritin in the presence or absence of a peptide; and
2) allowing the dissociated ferritin and the peptide to coexist in a buffer with a pH of 5.0 or more and 10.0 or less to generate the peptide-encapsulating ferritin, wherein
the peptide is composed of 3 to 19 amino acid residues and satisfies the following requirement:

a) $-10.2 \leq X \leq 5.9$ and $445 \leq Y \leq 2{,}524$

wherein X represents a charge of the peptide at pH 9, and Y represents a chemical formula weight of the peptide.

EFFECT OF THE INVENTION

The peptide-encapsulating ferritin according to the present invention can transport a certain peptide into a cell(s) without destroying a cell membrane(s). The peptide-encapsulating ferritin according to the present invention can avoid both immunogenicity and cytotoxicity caused by metabolites. Furthermore, the peptide-encapsulating ferritin according to the present invention can specifically and reproducibly transport a certain peptide into a transferrin receptor-presenting cell(s) (in particular, a cell(s) exhibiting a high expression level of a transferrin receptor).

BRIEF DESCRIPTION OF DRAWINGS

FIG. 1 is a diagram for confirmation of formation of a complex of ferritin (FTH) and peptide (PKC-F) by column chromatography.

FIG. 2 is a diagram showing a result of LC-MS analysis for a peptide (PKC-F)-encapsulated in ferritin (FTH).

FIG. 3 is a diagram showing a result of LC-MS analysis for a peptide (PKC-F).

FIG. 4 is a transmission electron microscope (TEM) image of a peptide (PKC-F)-encapsulating ferritin (FTH).

FIG. 5 is a profile representing comparison of peptide (PKC-F) non-encapsulating ferritin (FTH) and peptide (PKC-F)-encapsulating ferritin (FTH) in terms of their surface charges.

FIG. 6 is a set of graphs representing measurement results of incorporation of peptide (PKC-F) into transferrin receptor (TfR) presenting cell (SKBR-3 cell) by means of fluorescence activated cell sorting (FACS). The peptide (PKC-F) was used in an encapsulated state (as test object) within ferritin (FTH) and used alone (as control object).

FIG. 7 is a set of images representing measurement results of incorporation of peptide (PKC-F) into transferrin receptor (TfR) presenting cell (SKBR-3 cell) with use of two-photon excitation fluorescence microscope. The peptide (PKC-F) was used in an encapsulated state (as test object) within ferritin (FTH) and used alone (as control object).

FIG. 8 is a diagram for confirmation of formation of a complex of ferritin (FTH) and peptide (FAM-SV40) by column chromatography.

FIG. 9 is a set of graphs representing measurement results of incorporation of peptide (FAM-SV40) into transferrin receptor TfR presenting cell (SKBR-3 cell) by means of fluorescence activated cell sorting (FACS). The peptide (FAM-SV40) was used in an encapsulated state (as test object) within ferritin (FTH) and used alone (as control object).

FIG. 10 is a set of graphs representing comparison between transferrin receptor TfR presenting cells (SKBR-3 cell exhibiting high TfR expression level and HEK293E cell exhibiting low TfR expression level) in terms of incorporation of peptide (FAM-SV40) by means of fluorescence activated cell sorting (FACS).

FIG. 11 is a graph representing comparison between transferrin receptor TfR presenting cells (SKBR-3 cell exhibiting high TfR expression level and HEK293E cell exhibiting low TfR expression level) in terms of incorporation of peptide (FAM-SV40) by means of fluorescence activated cell sorting (FACS). The result of FIG. 10 is plotted in FIG. 11.

FIG. 12 is a graph representing the presence (A) of a MS peak of peptide (ClAc-FHC) derived from peptide (ClAc-FHC) encapsulating ferritin (FTH) in the case of performing the disassembly/reassembly process, and the absence (B) of a MS peak of the same in the case of not performing the disassembly/reassembly process.

FIG. 13 is a graph representing the presence of a MS peak of peptide (EGFR, cyloRGDfV, or FGF3-FP16) derived from peptide (EGFR, cyloRGDfV, or FGF3-FP16) encapsulating ferritin (FTH) in the case of performing the disassembly/reassembly process.

FIG. 14 is a graph representing the presence of a MS peak of peptide (Hymenistatin I, Ex10, or $\alpha v\beta6$) derived from peptide (Hymenistatin I, Ex10, or $\alpha v\beta6$) encapsulating ferritin (FTH) in the case of performing the disassembly/reassembly process.

FIG. 15 is a graph representing the presence of a MS peak of peptide (NRP-1-2, Murepavadin, or Pakti-L1) derived from peptide (NRP-1-2, Murepavadin, or Pakti-L1) encapsulating ferritin (FTH) in the case of performing the disassembly/reassembly process.

FIG. 16 is a graph representing the presence of a MS peak of peptide (NS, GRP78-3, or FBP0032) derived from peptide (NS, GRP78-3, or FBP0032) encapsulating ferritin (FTH) in the case of performing the disassembly/reassembly process.

FIG. 17 is a graph representing the presence of a MS peak of peptide (FBP0033) derived from peptide (FBP0033) encapsulating ferritin (FTH) in the case of performing the disassembly/reassembly process.

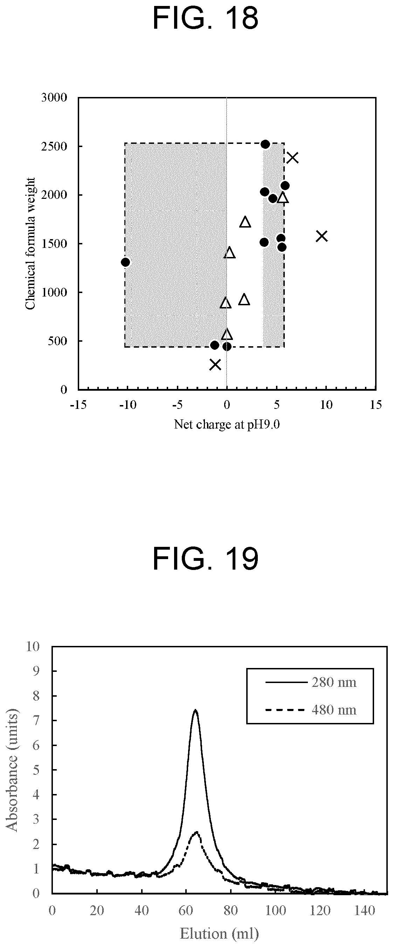

FIG. 18 is a set of plots representing correlation between charges of peptides at pH 9 and chemical formula weights of peptides in terms of capability and incapability of encapsulation of various peptides within ferritin (FTH) (three trials). Solid circle: Peptides with all three times of confirmation of reproducible encapsulation. Open triangles: Peptides with two times of confirmation of encapsulation or less. Crosses: Peptides without any confirmation of encapsulation. Namely, peptides satisfying requirement of a) $-10.2 \leq$ charge (X) of the peptide at pH $9 \leq 5.9$ and $445 \leq$ chemical formula weight (Y) of the peptide $\leq 2,524$, were successfully encapsulated in ferritin. Among peptides satisfying the above requirement, peptides satisfying requirement of b) $-10.2 \leq X \leq 0.0$ and $445 \leq Y \leq 2,524$ or c) $3.7 \leq X \leq 5.9$ and $445 \leq Y \leq 2,524$ were successfully reproducibly encapsulated in ferritin.

FIG. 19 is a diagram for confirmation of formation of a complex of ferritin (FTL) and peptide (PKC-F) by column chromatography.

EMBODIMENTS FOR CARRYING OUT THE INVENTION

The present invention provides a peptide-encapsulating ferritin including a peptide(s) that is composed of 3 to 19 amino acid residues and satisfies the following requirement:

a) $-10.2 \leq X \leq 5.9$ and $445 \leq Y \leq 2,524$.

(Here, X represents a charge of the peptide at pH 9. Y represents a chemical formula weight of the peptide.)

The amino acid constituting the peptide is not particularly limited as long as it is a compound having both an amino group and a carboxy group. For example, the amino acid constituting the peptide may be a naturally occurring amino acid (20 kinds of L-α-amino acid) constituting a protein, or an unnatural amino acid not constituting a protein. Examples of the naturally occurring amino acid include L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H), L-lysine (K), and glycine (G). As the unnatural amino acid, any L or D form of non-natural amino acid (e.g., α-amino acid, β-amino acid, and γ-amino acid) can be used. More specifically, examples of the non-natural amino acid include 2,4-diaminobutyric acid (Dab), 2,3-diaminopropionic acid (Dap), ornithine, homohistidine, homophenylalanine, cycloleucine, 1-aminocyclohexanecarboxylic acid, carboxycyclopropylamine, 2-aminocyclohexanecarboxylic acid, 1-aminocyclobutane carboxylic acid, tert-leucine, norvaline, norleucine, pyroglutamic acid, penicillamine, homo amino acid, 4-aminobutyric acid, 6-aminohexanoic acid, 7-aminoheptanoic acid, 4-amino-3-hydroxybutyric acid, 5-aminolevulinic acid, D-2-aminoadipic acid, and modified compounds of the above naturally occurring amino acids and unnatural amino acids (e.g., N-alkylated compounds such as N-methylated compounds, carbobenzoxyaminated compounds, tert-butoxycarbonylated compounds, 9-fluorenylmethyloxycarbonylated compounds and azidized compounds).

The peptide may be composed of only a natural amino acid(s), may be composed of only an unnatural amino acid(s), or may be composed of a mixture of a natural amino acid(s) and an unnatural amino acid(s). The peptide may be one in which an N-terminal amino group and a C-terminal carboxy group are protected or unprotected. Examples of a protecting group for the N-terminal amino group include an alkylcarbonyl group (acyl group) (e.g., acetyl group, propoxy group, and butoxycarbonyl group such as tert-butoxycarbonyl group), an alkyloxycarbonyl group (e.g., fluorenylmethoxycarbonyl group), an aryloxycarbonyl group, and an arylalkyl (aralkyl) oxycarbonyl group (e.g., benzyloxycarbonyl group). Examples of protecting group for the C-terminal carboxy group include a group capable of forming an ester or an amide. Examples of the group capable of forming an ester or an amide include alkyloxy groups (e.g., methyloxy, ethyloxy, propyloxy, butyloxy, pentyloxy, and hexyloxy), aryloxy groups (e.g., phenyloxy, and naphthyloxy), aralkyloxy groups (e.g., benzyloxy) and amino groups.

The peptide may be composed of an amide bond(s) and a carbon chain(s) in its main chain. Examples of the carbon chain include alkylenes (e.g., C1-C6 alkylene such as methylene, ethylene, propylene, butylene, pentylene, and hexylene), cycloalkylenes (e.g., C3-C8 cycloalkylene such as cyclopropylene, cyclobutylene, cyclopentylene, cyclohexylene, cycloheptylene, and cyclooctylene), and arylenes (e.g., C6-C12 arylene such as phenylene and naphthylene). Instead, the peptide may be formed to include a binding structure other than the amide bond and/or a chain structure other than the carbon chain in the main chain. Examples of the bonding structure other than the amide bond include a N-alkylamide bond (e.g., an amide bond having C1-C6 alkyl on a nitrogen atom(s) such as an N-methylamide bond or an N-ethylamide bond), an ester bond, an ether bond, a disulfide bond, a thioether bond, and a thioester bond. Examples of the chain structure other than the carbon chain include a structure including a heterocycle (e.g., oxazole, methyloxazole, thiazole, and triazole).

The peptide may have a linear, branched or cyclic structure. For example, the peptide having a cyclic structure may be obtained by cyclizing via an amide bond between an N-terminal amino acid residue and a C-terminal amino acid residue. A peptide containing a plurality of (e.g., two) cysteine residues can form a cyclic structure by forming a disulfide bond via a thiol group in the side chain of the cysteine residue. Even in the case of a peptide containing one cysteine residue, a cyclic structure can be formed by binding to either an N-terminal amino acid residue or a C-terminal amino acid residue.

The number of amino acid residues constituting the peptide is 3 to 19. The number of amino acid residues constituting the peptide may be preferably four or more. The number of amino acid residues constituting the peptide may be 18 or less, preferably 17 or less, and more preferably 16 or less. More specifically, the number of amino acid residues constituting the peptide may be 3 to 18, 3 to 17, 3 to 16, 4 to 18, 4 to 17, or 4 to 16.

In the present invention, as illustrated in FIG. 18, the peptide encapsulated in ferritin satisfies the following requirement.

a) $-10.2 \le X \le 5.9$ and $445 \le Y \le 2,524$;

(Here, X represents the charge of the peptide at pH 9, and Y represents the chemical formula weight of the peptide).

The value of the chemical formula weight of the peptide can be obtained as the total sum of products of atomic mass and the number of atoms based on the composition formula of the peptide.

The charge of the peptide at pH 9 can be evaluated with reference to ChemAxon (O. Toure et al., Oil Gas Sci. Technol. 2013, 68, 281-297) (See Example). When the evaluation using ChemAxon is not desired (for example, in the case of using an extremely special peptide with difficulty or lack of accuracy in the evaluation using ChemAxon), the charge may be evaluated according to experimental methods described in L. Settimo et. al., Pharm. Res. 2014, 31, 1082-1095.

Although any restriction of the present invention by theory is not desired, the peptide satisfying the above requirement can be encapsulated in ferritin for the following reasons.

(1) Reason why requirement of $-10.2 \leq X$ is desirable

Ferritin is strongly negatively charged in an aqueous solution having a pH higher than pH 5. Therefore, at the pH (e.g., pH 9) of the solution used for the encapsulation in ferritin, a molecule whose charge is from neutral to positive is considered to be easily encapsulated in ferritin, while a strongly negatively charged molecule is considered to be poorly encapsulated in ferritin. Therefore, the strongly negatively charged peptide with $-10.2 > X$ is considered to be poorly encapsulated in ferritin because of electrostatic repulsion against ferritin. On the other hand, the peptide with $-10.2 \leq X$ can be encapsulated in ferritin, because of stronger forces for forming the cage-like super molecular structure than the repulsion due to the negative charges between the peptide and ferritin.

(2) Reason why requirement of $X \leq 5.9$ is desirable

The peptide charged too positively is adsorbed on interior and exterior of ferritin due to electrostatic interaction, possibly preventing ferritin from forming the cage-like structure, thereby unlikely to be encapsulated. In addition, the strongly positively charged peptide can be easily aggregated to form its particle with such a size as not to be encapsulated in ferritin, thereby unlikely to be encapsulated.

(3) Reason why requirement of $445 \leq Y$ is desirable

The peptide with too small chemical formula weight can be easily flowed out by diffusion from the interior of ferritin while ferritin forms the cage-like structure, thereby unlikely to be encapsulated.

(4) Reason why requirement of $Y \leq 2,524$ is desirable

The diameter of the ferritin internal cavity is 7 nm. Bulky molecules with a large chemical formula weight are considered to be poorly encapsulated in ferritin.

Preferably, from the viewpoint of improvement in the reproducibility of the encapsulation of the peptide in ferritin, or the like, the peptide satisfies the following requirement:

b) $-10.2 \leq X \leq 0.0$ and $445 \leq Y \leq 2,524$, or c) $3.7 \leq X \leq 5.9$ and $445 \leq y \leq 2,524$.

(5) Reason why $0.0 < X < 3.7$ is excluded

A poorly charged peptide with a substantially large chemical formula weight can be aggregated itself to form a particle with such a size as not to be encapsulated in ferritin, and is considered to be poorly encapsulated in ferritin.

Ferritin derived from various animals can be used as ferritin according to the present invention. Examples of the animal from which the ferritin is derived include mammals and avian species (e.g., chicken), but mammals are preferable. Examples of mammals include primates (e.g., humans, monkeys, and chimpanzees), rodents (e.g., mice, rats, hamsters, guinea pigs, and rabbits), and livestock and working mammals (e.g., cattle, pigs, sheep, goats, and horses).

It is known that there are two kinds of monomers, ferritin H chain and L chain as ferritin monomers constituting ferritin in animals such as humans, and that ferritin is a multimer (mixture of H chain and L chain, in many cases) formed of 24 monomers. Therefore, in the present invention, ferritin formed of ferritin H chains only, ferritin formed of ferritin L chains only, and ferritin formed of a mixture of ferritin H chain(s) and L chain(s) can be used.

Either a naturally occurring ferritin monomer or a genetically modified ferritin monomer capable of forming 24-mer can be used as the ferritin monomer. For example, the genetically modified ferritin monomer may be modified in a flexible linker region between $\alpha$-helices among six $\alpha$-helices constituting the ferritin monomer. Examples of such a genetically modified ferritin monomer include a ferritin monomer with a functional peptide inserted into a flexible linker region between first and second, second and third, third and fourth, fourth and fifth, or fifth and sixth $\alpha$-helices counted from N-terminus of the ferritin monomer including six $\alpha$-helices (e.g., US Patent Application Publication No. 2016/0060307; Jae Og Jeon et al., ACS Nano (2013), 7 (9), 7462-7471; Sooji Kim et al., Biomacromolecules (2016), 17 (3), 1150-1159; Young Ji Kang et al., Biomacromolecules (2012), 13 (12), 4057-4064). For example, first to sixth $\alpha$-helices of six $\alpha$-helices counted from N-terminus of a human ferritin H chain (SEQ ID NO: 1) and a human ferritin L chain (SEQ ID NO: 2) are listed in Table 1. The flexible linker region of the human ferritin H chain (SEQ ID NO: 1) refers to the flexible linker region between first and second (region composed of 43th to 49th amino acid residues), the flexible linker region between second and third (region composed of 78th to 96th amino acid residues), the flexible linker region between third and fourth (region composed of 125th to 127th amino acid residues), the flexible linker region between fourth and fifth (position at 138th amino acid residue), or the flexible linker region between fifth and sixth (region composed of 160th to 164th amino acid residues). The flexible linker region of the human ferritin L chain (SEQ ID NO: 2) refers to the flexible linker region between first and second (region composed of 38th to 45th amino acid residues), the flexible linker region between second and third (region composed of 74th to 92th amino acid residues), the flexible linker region between third and fourth (region composed of 121st to 123rd amino acid residues), the flexible linker region between fourth and fifth (position at 134th amino acid residue), or the flexible linker region between fifth and sixth (region composed of 155th to 159th amino acid residues).

Table A. Positions of α-helices in ferritin

| α-helix | Positions of amino acid residues in amino acid sequence | |
|---|---|---|
| | Human ferritin | |
| | H chain (SEQ ID NO: 1) | L chain (SEQ ID NO: 2) |
| First | 15-42 | 11-37 |
| Second | 50-77 | 46-73 |
| Third | 97-124 | 93-120 |
| Fourth | 128-137 | 124-133 |
| Fifth | 139-159 | 135-154 |
| Sixth | 165-174 | 160-170 |
| α-helices are classified according to Int J Mol Sci. 2011; 12(8): 5406-5421. | | |

The genetically modified ferritin monomer may be modified in the N-terminus region and/or C-terminus region thereof. The N-terminus region of the ferritin monomer is exposed on a surface of the multimer, while the C-terminus thereof is unable to be exposed on the surface. Therefore, the peptide moiety added to the N-terminus of the ferritin monomer is exposed on the surface of the multimer, and is capable of interacting with a target material present outside the multimer (e.g., WO2006/126595). On the other hand, the C-terminus of the ferritin monomer can interact with the organic compound inside the ferritin cavity by modifying its amino acid residue (e.g., Y. J. Kang, Biomacromolecules. 2012, vol. 13 (12), 4057). Examples of such a genetically modified ferritin monomer include a ferritin monomer with the functional peptide added to the N-terminus or C-terminus.

Preferably, ferritin is human ferritin from the viewpoint of clinical application. Human ferritin formed of human ferritin H chains only, human ferritin formed of human ferritin L chains only, and human ferritin formed of a mixture of human ferritin H chain(s) and L chain(s) can be used as human ferritin.

Preferably, the human ferritin H chain may be as follows:

(A1) a protein comprising the amino acid sequence of SEQ ID NO: 1;
(B1) a protein comprising an amino acid sequence including one or several modification(s) of an amino acid residue(s) selected from the group consisting of substitution, deletion, insertion and addition of the amino acid residue(s) in the amino acid sequence of SEQ ID NO: 1, the protein capable of forming a 24-mer; or
(C1) a protein comprising an amino acid sequence having 90% or more identity to the amino acid sequence of SEQ ID NO: 1, the protein capable of forming a 24-mer.

Preferably, the human ferritin L chain may be as follows:

(A2) a protein comprising the amino acid sequence of SEQ ID NO: 2;
(B2) a protein comprising an amino acid sequence including one or several modification(s) of an amino acid residue(s) selected from the group consisting of substitution, deletion, insertion and addition of the amino acid residue(s) in the amino acid sequence of SEQ ID NO: 2, the protein capable of forming a 24-mer; or
(C2) a protein comprising an amino acid sequence having 90% or more identity to the amino acid sequence of SEQ ID NO: 2, the protein capable of forming a 24-mer.

In the above (B1) and (B2), one or several amino acid residue(s) can be modified by one, two, three or four kinds of modifications selected from the group consisting of deletion, substitution, addition and insertion of amino acid residues. The modification of the amino acid residues may be introduced into one region in the amino acid sequence, or may be introduced into a plurality of different regions. The term "one or several" represents the number that is selected not to impair activities of the protein. The number represented by the term "one or several" refers to 1 to 50, for example, preferably 1 to 40, more preferably 1 to 30, still more preferably 1 to 20, and particularly preferably 1 to 10 or 1 to 5 (e.g., 1, 2, 3, 4 or 5).

In the above (C1) and (C2), the identity % of the protein can be determined by algorithm blastp. More specifically, the identity % of the polypeptide can be determined by using default settings of Scoring Parameters (Matrix: BLOSUM62; Gap Costs: Existence = 11 Extension = 1; Compositional Adjustments: Conditional compositional score matrix adjustment) in the algorithm blastp provided by National Center for Biotechnology Information (NCBI).

The position of the amino acid residue to which the mutation is introduced in the amino acid sequence is apparent to a person skilled in the art, but may be determined with further reference to the sequence alignment. Specifically, a person skilled in the art can (1) compare a plurality of amino acid sequences, (2) reveal a relatively conserved region and a region that is not relatively conserved, and (3) then predict a region capable of playing important roles for functions and a region incapable of playing important roles for functions from the relatively conserved region and the region that is not relatively conserved, respectively, for recognition of correlations between structures and functions. Therefore, a person skilled in the art can determine the position to which the mutation should be introduced in the amino acid sequence by utilizing the sequence alignment, as well as the position of the amino acid residue to which the mutation should be introduced in the amino acid sequence by combination use of known secondary and tertiary structure information. Preferably, the abovementioned flexible linker region, and N terminus and C terminus regions can be used as sites for introducing the mutation.

When the amino acid residue is mutated by substitution, the substitution of the amino acid residue may be a conservative substitution. The term "conservative substitution" used in this description refers to replacing a predetermined amino acid residue with an amino acid residue having an analogous side chain. Families of the amino acid residues with analogous side chains are known in the relevant field. Examples of such families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine and cysteine), amino acids with non-polar side chains (e.g., alanine, valine, leucine, isoleucine, proline, phenylalanine, methionine and tryptophan), amino acids with β-branched amino acids (e.g., threonine, valine and isoleucine), amino acids with aromatic side chains (e.g., tyrosine, phenylalanine, tryptophan and histidine), amino acids with hydroxy group (e.g., alcoholic and phenolic)-containing side chains (e.g., serine, threonine and tyrosine), and amino acids with sulfur-containing side chains (e.g., cysteine and methionine). Preferably, the conservative substitutions of amino acids may be substitution between aspartic acid and glutamic acid, substitution among arginine, lysine and histidine, substitution between tryptophan and phenylalanine, substitution between phenylalanine and valine, substitution among leucine, isoleucine and alanine, and substitution between glycine and alanine.

The genetically modified ferritin monomer may be a genetically modified ferritin monomer containing a functional peptide. As the functional peptide, it is possible to use a peptide that allows an arbitrary function to be added to the aimed protein when fused to the aimed protein. Examples of such a functional peptide include a peptide capable of binding to a biological organic molecule, a protease-degrading peptide, a stabilizing peptide, and a cell-permeable peptide.

Examples of biological organic molecules include proteins (e.g., oligopeptide or polypeptide), nucleic acids (e.g., DNA, RNA, nucleosides, nucleotides, oligonucleotides or polynucleotides), saccharides (e.g., monosaccharides, oligosaccharides or polysaccharides) and lipids. The biological organic molecule may also be a cell surface antigen (e.g., a cancer antigen, a heart disease marker, a diabetes marker, a neurological disease marker, an immune disease marker, an inflammatory marker, a hormone, or an infectious disease marker). The biological organic molecule may also be a disease antigen (e.g., a cancer antigen, a heart disease marker, a diabetes marker, a neurological disease marker, an immune disease marker, an inflammatory marker, a hormone, or an infectious disease marker). As the peptide capable of binding to such a biological organic molecule, various peptides have been reported as follows: for example, peptides capable of binding to protein (see e.g., F. Danhier et al., Mol. Pharmaceutics, 2012, vol. 9, No. 11, p. 2961; C-H. Wu et al., Sci. Transl. Med., 2015, Vol. 7, No. 290, 290-91; L. Vannucci et al., Int. J. Nanomedicine 2012, Vol. 7, p. 1489; J. Cutrera et al., Mol. Ther. 2011, Vol. 19 (8), p. 1468; R. Liu et al., Adv. Drug Deliv. Rev. 2017, Vol. 110-111, p. 13); peptides capable of binding to nucleic acid (see e.g., R. Tan et al., Proc. Natl. Acad. Sci. USA, 1995, vol. 92, p. 5282; R. Tan et al., Cell, 1993, vol. 73, p 1031; R. Talanian et al., Biochemistry 1992, Vol. 31, p. 6871); peptides capable of binding to saccharide (see e.g., K. Oldenburg et al., Proc. Natl. Acad. Sci. USA, 1992, vol. 89, No. 12, p. 5393-5397; K. Yamamoto et al., J. Biochem., 1992, vol. 111, p. 436; A. Baimiev et al., Mol. Biol. (Moscow), 2005, vol. 39, No. 1, p. 90); and peptides capable of binding to lipid (see e.g., O. Kruse et al., B Z. Naturforsch., 1995, Vol. 50c, p. 380; O. Silva et al., Sci. Rep., 2016, Vol. 6, 27128; A. Filoteo et al., J. Biol. Chem., 1992, vol. 267, No. 17, p. 11800).

When a protease-degrading peptide is used as the functional peptide, examples of the protease include cysteine protease such as caspase and cathepsin (D. McIlwain1 et al., Cold Spring Harb Perspect Biol., 2013, vol. 5, a008656; V. Stoka et al., IUBMB Life. 2005, vol. 57, No. 4-5 p. 347), collagenase (G. Lee et al., Eur J Pharm Biopharm., 2007, vol. 67, No. 3, p. 646), thrombin and Xa factor (R. Jenny et al., Protein Expr. Purif., 2003, vol. 31, p. 1; H. Xu et al., J. Virol., 2010, vol. 84, No. 2, p. 1076), and a virus-derived protease (C. Byrd et al., Drug Dev. Res., 2006, vol. 67, p. 501).

As the protease-degrading peptide, various peptides have been reported (see e.g., E. Lee et al., Adv. Funct. Mater., 2015, vol. 25, p. 1279; G. Lee et al., Eur J Pharm Biopharm., 2007, vol. 67, no. 3, p. 646; Y. Kang et al., Biomacromolecules, 2012, vol. 13, No. 12, p. 4057; R. Talanian et al., J. Biol. Chem., 1997, vol. 272, p. 9677; Jenny et al., Protein Expr. Purif., 2003, vol. 31, p. 1; H. Xu et al., J. Virol., 2010, vol. 84, No. 2, p. 1076). Therefore, in the present invention, such peptides or mutant peptides thereof (e.g., mutation such as conservative substitutions of one, two, three, four or five amino acid residues), or such peptides or peptides with one or more amino acid sequences of the mutant peptides can be used as the protease-degrading peptide.

As the stabilizing peptide, various peptides have been reported (see e.g., X. Meng et al., Nanoscale, 2011, Vol. 3, No. 3, P. 977; E. Falvo et al., Biomacromolecules, 2016, vol. 17, No. 2, p. 514). Therefore, in the present invention, such peptides or mutant peptides thereof (e.g., mutation such as conservative substitutions of one, two, three, four or five amino acid residues), or such peptides or peptides with one or more amino acid sequences of the mutant peptides can be used as the stabilizing peptide.

As the cell-permeable peptide, various peptides have been reported (see e.g., Z. Guo et al. Biomed. Rep., 2016, vol. 4, No. 5, p. 528). Therefore, in the present invention, such peptides or mutant peptides thereof (e.g., mutation such as conservative substitutions of one, two, three, four or five amino acid residues), or such peptides or peptides with one or more amino acid sequences of the mutant peptides can be used as the cell-permeable peptide.

The functional peptide is preferably the peptide capable of binding to the biological organic molecule. The peptide capable of binding to biological organic molecule is preferably the peptide capable of binding to protein.

The present invention also provides a peptide transport agent including the peptide-encapsulating ferritin described above.

The peptide transport agent according to the present invention transports the peptide in the peptide-encapsulating ferritin to the interior of the transferrin receptor-presenting cell by the interaction between the ferritin and the transferrin receptor-presenting cell. Examples of the transferrin receptor-presenting cell include cells present in tissues and regions of epidermal basal layer, pancreatic endocrine tissue, brain pituitary gland, placenta, cerebral cortex, corpus striatum, kidney proximal renal tubular, esophagus, endocervical canal, gastric mucosa epithelium, chest epithelium and the like, as well as cells such as erythrocytes, hemocytoblasts, brain capillary endothelial cells, hepatocytes, Kupffer cell and hippocampal neuron. A cell from which any mammal is derived can be used as the transferrin receptor presenting cell. Examples of such mammals include primates (e.g., humans, monkeys, and chimpanzees), rodents (e.g., mice, rats, hamsters, guinea pigs, and rabbits), and livestock and working mammals (e.g., cattle, pigs, sheep, goats, and horses). From the viewpoint of clinical application, the mammal is preferably a human.

The peptide transport agent according to the present invention can transport the peptide to the interior of whole transferrin receptor presenting cell, and can transport the peptide more specifically and reproducibly to the interior of the cell with a high transferrin receptor expression level. Therefore, the transferrin receptor presenting cell is preferably the cell with a high transferrin receptor expression level. Examples of the cell with a high transferrin receptor expression level include, among the transferrin receptor presenting cell described above, cells present in tissues and regions of epidermal basal layer, pancreatic endocrine tissue, brain pituitary gland, placenta and the like, as well as cells such as erythrocytes, hemocytoblasts, brain capillary endothelial cells, hepatocytes and Kupffer cell.

In a specific embodiment, the transferrin receptor presenting cell may be a cell that can be a cause of a specific disease. Such a specific disease may include cancer, leukemia and malignant lymphoma, but is preferably cancer. Examples of the cancer include breast cancer, pancreatic cancer, ovarian cancer, uterine cancer, lung cancer (e.g., squamous epithelial cancer, non-small cell cancer such as adenocarcinoma and large cell cancer, and small cell cancer), gastrointestinal cancer (e.g., gastric cancer, small intestine cancer, colorectal cancer, and rectal cancer), pancreatic cancer, kidney cancer, thymus cancer, spleen cancer, thyroid cancer, adrenal cancer, prostate cancer, bladder cancer, bone cancer, skin cancer, brain tumor, and melanoma. The cancer is preferably breast cancer. Therefore, the peptide transport agent of the present invention is useful for the prevention or treatment of such specific diseases.

The present invention also provides a method for producing the peptide-encapsulating ferritin described above. The method includes the following (1) and (2):

> 1) leaving ferritin in a buffer having a pH of 3.0 or less to dissociate ferritin (disassembling process); and
> 2) allowing the dissociated ferritin and the peptide to coexist in a buffer having a pH of 5.0 or more and 10.0 or less to produce the peptide-encapsulating ferritin (reassembling process).

In the above 1), ferritin is allowed to stand in the buffer having a pH of 3.0 or less to allow ferritin (24-mer) to be dissociated into the ferritin monomers. The standing of ferritin in the buffer can be carried out in the presence or absence of the peptide. When the ferritin is allowed to stand in the buffer in the absence of the peptide, the peptide is added to the buffer before the above 2).

Any buffer capable of being adapted to acidic conditions with a pH of 3.0 or less can be used as the buffer. Examples of such buffers include a glycine buffer; a citrate buffer; an acetate buffer; a phosphate buffer; a carbonate buffer; a borate buffer; a tartrate buffer; and an MES (2-morpholinoethanesulfonic acid) buffer.

The pH of the buffer used in 1) is preferably 2.8 or less, more preferably 2.6 or less, and still more preferably 2.5 or less. The pH can be measured at 25°C using a pH meter (LAQUA, F-72, Horiba) and a pH electrode (9680S-10D).

The standing time of ferritin in the buffer having a pH of 3.0 or less is not particularly limited as long as it is the time required for the disassembly of ferritin. More specifically, such a time is different depending on conditions such as pH, but is 1 minute to 10 hours, for example, preferably 2 minutes to 5 hours, more preferably 3 minutes to 2 hours, still more preferably 4 minutes to 1 hour, and particularly preferably 5 to 30 minutes, from the viewpoint of the efficiency of producing the peptide-encapsulating ferritin, the time saving for producing the peptide-encapsulating ferritin and the like.

After the completion of 1), the operation 2) can be carried out by exchanging the buffer or adding a buffer or a base. For example, the exchange of the buffer can be performed by neutralizing and leaving the buffer to stand after the operation of 1), then collecting a supernatant containing the dissociated ferritin and the peptide, and then mixing the supernatant with the buffer having a pH of 5.0 or more and 10.0 or less. Examples of the buffer having a pH of 5.0 or more and 10.0 or less includes a Tris buffer, an HEPES buffer, a phosphate buffer, a borate buffer, a citrate buffer, a carbonate buffer, and a glycine buffer.

The pH of the buffer used in 2) is preferably 5.0 or more and 9.5 or less, more preferably 5.0 or more and 9.2 or less, and still more preferably 5.0 or more and 9.0 or less.

The time for leaving the dissociated ferritin and the peptide to stand in the buffer is not particularly limited as long as it is the time required for the reassembly of ferritin. More specifically, the time is different depending on conditions such as pH, but is 10 minutes to 72 hours, for example, preferably 20 minutes to 48 hours, and more preferably 30 minutes to 24 hours, from the viewpoint of the efficiency of producing the peptide-encapsulating ferritin, the time saving for producing the peptide-encapsulating ferritin and the like.

EXAMPLES

Next, the present invention is described more in detail with reference to Examples, but the present invention is not limited to the following Examples.

<Example 1: Preparation of ferritin>

Total synthesis was carried out for a DNA encoding a human-derived ferritin H chain (FTH (SEQ ID NO: 1)). PCR was carried out using the total- synthesized DNA as a template as well as the following primers: 5'-GAAGGAGATATACATATGACGACCGCGTCCACCTCG-3' (SEQ ID NO: 3) and 5'-CTCGAATTCGGATCCTTAGCTTTCATTATCACTGTC-3' (SEQ ID NO: 4). PCR was carried out using pET20 (Merck KGaA) as a template as well as the following primers: 5'-TTTCATATGTATATCTCCTTCTTAAAGTTAAAC-3' (SEQ ID NO: 5) and 5'-TTTGGATCCGAATTCGAGCTCCGTCG-3' (SEQ ID NO: 6). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTH) on which a gene encoding FTH was loaded.

Next, Escherichia coli BL21 (DE3) into which the constructed pET20-FTH was introduced was cultured in 100 mL of an LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with a 50 mM Tris-HCl buffer (pH 8.0). Then, an aimed protein was separated and purified by applying a concentration gradient of the salt from 0 mM to 500 mM NaCl in a 50 mM Tris-HCl buffer (pH 8.0). The solvent of the solution containing the protein was replaced with a 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with a 10 mM Tris-HCl buffer (pH 8.0) to separate and purify FTH by size. The solution containing FTH was concentrated by centrifugal ultrafiltration using Vivaspin 20-100K (GE Healthcare Inc.), and then concentration of the contained protein was determined using a protein assay CBB solution (Nacalai Tesque, Inc.) in terms of bovine albumin as a standard. As a result, a 1 mL solution containing 5 mg/mL of FTH was obtained from 100 mL of the culture solution.

pH was measured at 25°C using a pH meter (LAQUA, F-72, Horiba) and a pH electrode (9680S-10D).

<Example 2: Study of encapsulation of modified fluorescent peptide (1)>

For peptide transport using ferritin, construction of FTH into which a fluorescent dye fluorescein (FAM)-modified peptide (5(6)-FAM-RFARKGALRQKNVHEVKN (SEQ ID NO: 9), PKC-F, Chemical formula weight 2,524 in Table 3) was encapsulated, was attempted.

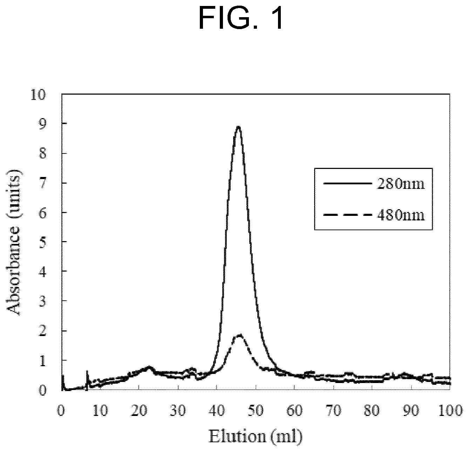

Of a 50 mM glycine hydrochloride buffer (pH 2.3) that contains FTH (chemical formula weight: 509,421) at a final concentration of 5 mg/mL and the peptide at a final concentration of 0.5 mM, 0.5 mL was left to stand at room temperature for 15 minutes. Next, 50 μL of a 1 M Tris hydrochloride buffer (pH 9.0) was added for neutralization, and the resulting solution was left to stand at room temperature for 3 hours. After the standing, the resulting solution was centrifuged (at 15,000 rpm for one minute). Then, the supernatant was collected and the volume was increased up to 3 mL with a 10 mM Tris hydrochloride buffer (pH 8). The resulting solution was injected into the HiPrep 26/60 Sephacryl S-300 HR column (GE Healthcare Inc.) equilibrated with a 10 mM Tris-HCl buffer (pH 8.0) to separate and purify 24-mer FTH by size at a flow rate of 1.3 mL/minute. The purified ferritin was analyzed using a HiPrep 16/60 Sephacryl S-300 HR column (GE Healthcare Inc.) equilibrated with a 10 mM Tris-HCl buffer (pH 8.0) at a flow rate of 0.5 mL/minute and confirmed as the absorbance at 480 nm unobservable from FTH only, at the same position as that of a peak of FTH. It reveals that FTH and the peptide form a complex (FIG. 1).

The solution containing the FTH was concentrated to 0.25 mL by centrifugal ultrafiltration using Vivaspin 20-100K (GE Healthcare Inc.). LC-MS analysis revealed that the peptide was contained in the solution. For the LC-MS analysis, 50 μL of a 1 M glycine hydrochloride buffer (pH 2.3) was added to 50 μL of the sample solution and then the resulting solution was left to stand at room temperature for 15 minutes. To the solution 900 μL of ethanol was added. Next, the resulting solution was stirred vigorously and then centrifuged (at 15,000 rpm for five minutes) to collect its supernatant. The supernatant was analyzed using LCMS-2020 (Shimadzu Corporation) as LC-MS, Inertsil (Registered trademark) ODS-3 with a particle diameter of 2 μm and 2.1 mm × 50 mm (GL science) as a column. Into the column, 10 μL of the sample was injected, and eluded at a flow rate of 0.2 mL/min for 10 minutes such that a mixing ratio of a solution A (0.1% formic acid / 99.9% acetonitrile) and a solution B (50% water / 50% acetonitrile) was from 95:5 to 95:5. The concentration of the contained protein was determined using a protein assay CBB solution (Nacalai Tesque, Inc.) in terms of bovine albumin as a standard. The concentration of the encapsulated peptide was determined based on the absorbance at 480 nm of the fluorescent dye FAM to which PKC-F is modified.

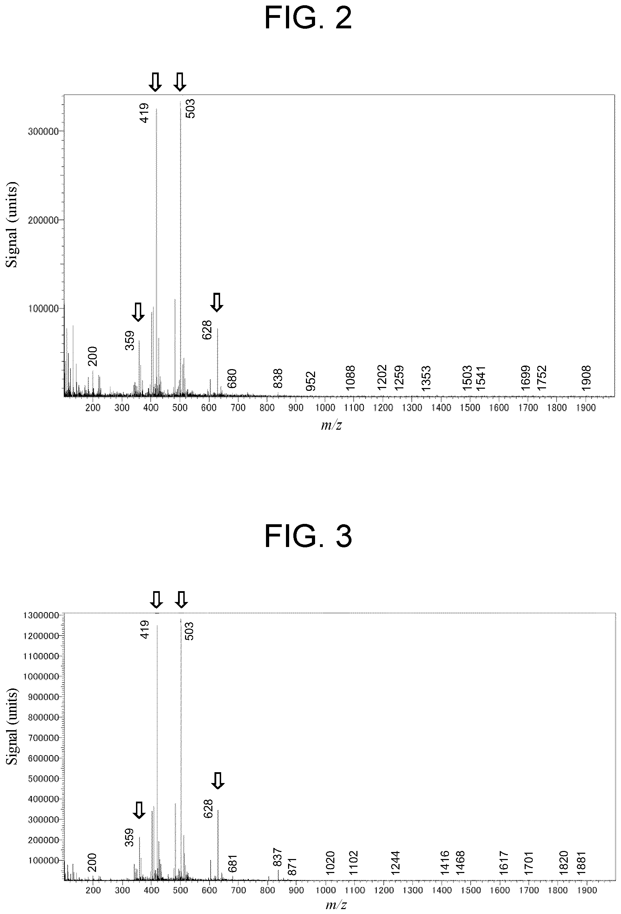

According to results of the LC-MS analysis, the retention time was 3.9 minutes for both the peptide (FIG. 2) encapsulated in ferritin and the standard peptide (FIG. 3) and MS spectra were also identical, demonstrating that PKC-F was encapsulated by the ferritin encapsulation operation without being decomposed. Furthermore, 0.25 mL of the solution containing 4.3 mg/mL (8.5 μM) of FTH and 0.18 mg/mL (72 μM) of the peptide was obtained. The molar ratio indicates that 8.5 molecules of PKC-F were encapsulated in one molecule of ferritin on average.

<Example 3: Shape of ferritin encapsulating peptide modified with fluorescent dye>

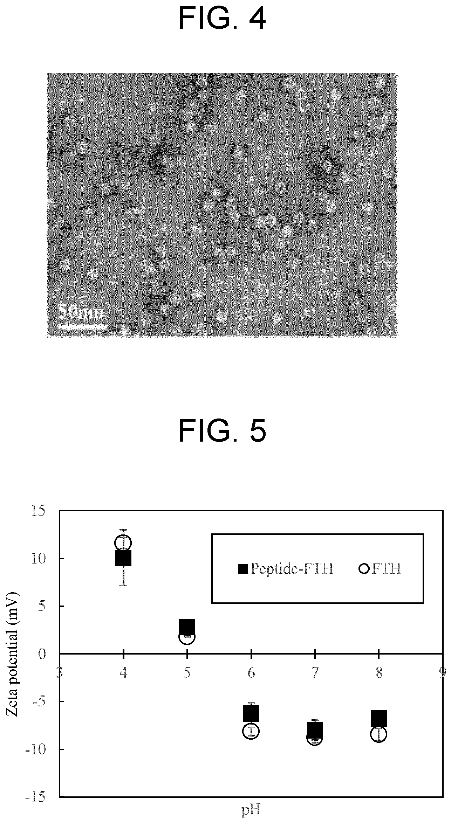

As illustrated in FIG. 4, a cage-like structure of the purified PKC-F encapsulating FTH was confirmed from an image of transmission electron microscopy (TEM) with 3% phosphotungstic acid staining. The peptide-encapsulating FTH has a diameter of 12 nm, same size as that of naturally occurring human ferritin. It reveals that the peptide is encapsulated without substantially damaging high-order structure of ferritin.

The PKC-F encapsulating FTH complex was suspended in a 50 mM phosphate buffer (pH 6 or 7), an acetate buffer (pH 4 or 5) or a Tris-hydrochloride buffer (pH 8) to have a final concentration of 0.1 g/L as a FTH amount. The surface charge at 25°C was measured for the buffer with use of Zetasizer Nano ZS (Malvern Panalytical Ltd.). The measurement was carried out using a DTS 1070 cell filled with 750 μL of a sample with the following settings: Material setting (RI: 1.450, Absorption: 0.001), Dispersant setting (Temperature: 25°C, Viscosity: 0.8872 cP, RI: 1.330, Dielectric constant: 78.5) and Smoluchowski model (F $\kappa$a value: 1.50). As a result, the FTH without the peptide encapsulation has a comparable surface charge to that of the PKC-F encapsulating FTH complex, indicating that the peptide was not adsorbed on the FTH surface to form the complex (FIG. 5).

<Example 4: Cell incorporation test with use of ferritin encapsulating fluorescent dye modified peptide (1) >

The obtained PKC-F encapsulating FTH was used for evaluating transportability of PKC-F to the interior of cells by ferritin.

A human breast cancer derived SKBR-3 cell (20,000 cell/well, 96-well plate) was added to 100 μL media that were produced by adding the PKC-F encapsulating FTH to evaluation media (Opti-MEM™ (Thermo Fisher Scientific Inc.) + 1% non-essential amino acid solution (Thermo Fisher Scientific Inc.) + 1% penicillin- streptomycin (Nacalai Tesque, Inc.)) at final concentrations of 100 nM (PKC-F concentration 0.86 μM), 200 nM (PKC-F concentration 1.72 μM) and 400 nM (PKC-F concentration 3.44 μM), respectively, and then cultured at 37°C. The transferrin receptor TfR was expressed in the SKBR-3 cell. As controls, a SKBR-3 cell was cultured in the same way in media to which the peptide without FTH encapsulation was added at a final concentration of 0.86 μM, 1.72 μM or 3.44 μM. After the cell culture for 24 hours for each, the resulting solution was washed twice with 100 μL of a phosphate buffer saline solution and left to stand in 50 μL of Trypsin-EDTA (Sigma-Aldrich, Inc.) at 37°C for 10 minutes. After that 100 μL of an Opti-MEM™ medium was added, and the cells were transferred to a plate for fluorescence-activated cell sorting (FACS), and then subjected to centrifugal separation at 400 × g for 5 minutes. The cells for each were suspended in a FACS buffer (Attune™ Focusing Fluid, Thermo Fisher Scientific Inc.), and analyzed by FACS (Attune NxT, Thermo Fisher Scientific Inc.).

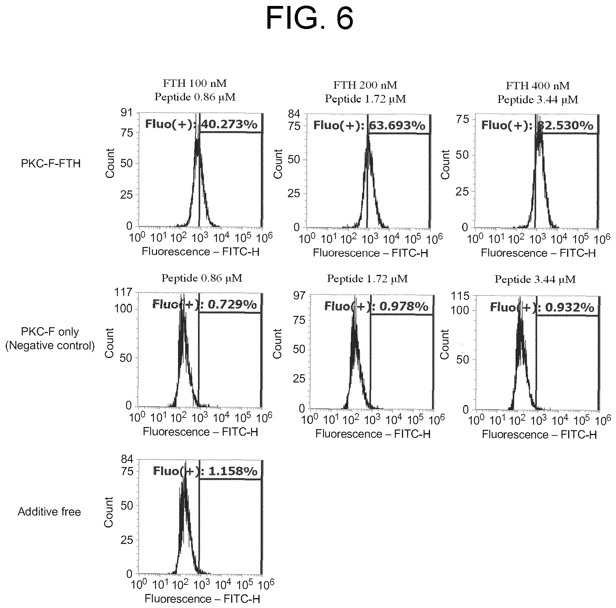

As a result, it is confirmed that the fluorescence intensity varied depending on the concentration of the PKC-F encapsulating FTH (FIG. 6), suggesting increase in the amount of incorporation into cells dependent on the concentration.

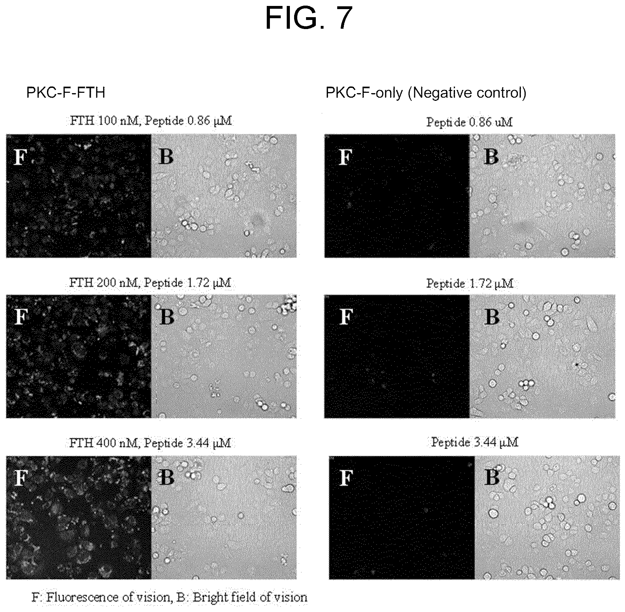

The cells prepared by the same condition were observed with two-photon excitation fluorescence microscope (CQ-1, Yokogawa Electric Corporation), confirming that the fluorescence at the interior of cells was observable only for the PKC-F encapsulation, and that the incorporation of the PKC-F encapsulating FTH into the cells was concentration dependent (FIG. 7). On the other hand, the transport to the interior of cells were not confirmed for PKC-F without the PKC-F encapsulation.

The above results indicated that the encapsulation in the FTH enables the peptide to be transported into the TfR presenting cells.

<Example 5: Investigation of encapsulation of peptide modified with florescent dye (2)>

For investigation of versatility in the peptide transport with use of ferritin, encapsulation of peptides other than PKC-F into ferritin for the purpose of transporting them into cells was attempted.

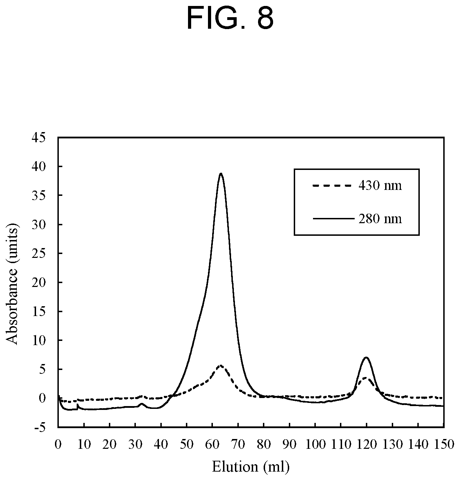

First, the construction of FTH encapsulating two kinds of fluorescent dye-modified peptides [5(6)-FAM-CGGPKKKRKVG (SEQ ID NO: 10), FAM-SV40, chemical formula weight 1,516, and 5(6)-FAM-CGGKRPAAIK KAGQAKKKKG (SEQ ID NO: 11), FAM-Np, chemical formula weight 384] was attempted. 0.5 mL of a 50 mM glycine hydrochloride buffer (pH 2.3) that contains FTH (chemical formula weight 509,421) at a final concentration of 5 mg/mL and a peptide at a final concentration of 0.5 mM, was left to stand at room temperature for 15 minutes. Next, 50 $\mu$L of a 1 M Tris hydrochloride buffer (pH 9.0) was added for neutralization, and the resulting solution was left to stand at room temperature for 3 hours. After the standing, the resulting solution was centrifuged (at 15,000 rpm for one minute). Then, the supernatant was collected and diluted with a 10 mM Tris hydrochloride buffer (pH 8) up to 3 mL. The resulting solution was injected into the HiPrep 16/60 Sephacryl S-300 HR column (GE Healthcare Inc.) equilibrated with D-PBS (-) (Fujifilm Wako Pure Chemical Corporation) to separate and purify 24-mer FTH by size at a flow rate of 0.5 mL/minute. The absorbance at 430 nm was confirmed at the same elusion position as that of ferritin 24-mer, for ferritin that was used for the operation to encapsulate FAM-SV40 therein. It was indicated that FAM-SV40 was encapsulated in ferritin (FIG. 8). However, the absorbance at 480 nm was not confirmed for ferritin that was used for the operation to encapsulate FAM-Np therein, revealing that FAM-Np was not encapsulated. It suggests the presence of peptide poorly encapsulated in ferritin depending on a different physical property and the like.

Next, the peptide amount of FAM-SV40 encapsulated in purified ferritin was quantified based on the 480 nm absorbance and by CBB staining with use of ferritin-amount-albumin as a standard.

As a result, 0.3 mL of the solution containing 6.3 mg/mL (12 $\mu$M) of FTH and 0.09 mg/mL (30 $\mu$M) of FAM-SV40 peptide was obtained. According to the molar ratio, 2.5 molecules of FAM-SV40 were encapsulated in one molecule of ferritin on average.

<Example 6: Cell incorporation test with use of ferritin encapsulating fluorescent dye modified peptide (2)>

The cell incorporation of the obtained FAM-SV40 encapsulating FTH was evaluated. SKBR-3 cell (20,000 cell/well, 96-well plate) was added to 100 $\mu$L media that were produced by adding the FAM-SV40 encapsulating FTH to evaluation media (Opti-MEM™ (Thermo Fisher Scientific Inc.) + 1% non-essential amino acid solution (Thermo Fisher Scientific Inc.) + 1% penicillin- streptomycin (Nacalai Tesque, Inc.)) at final concentrations of 100 nM (FAM-SV40 concentration 250 nM), 200 nM (FAM-SV40 concentration 500 nM) and 400 nM (FAM-SV40 concentration 1000 nM), respectively, and then cultured at 37°C. As controls, SKBR-3 cell was cultured in the same way in media to which the peptide without FTH encapsulation was added at final concentrations of 250 nM, 500 nM and 1,000 nM, respectively. After the cell culture for 24 hours for each, the resulting solution was washed twice with 100 $\mu$L of a phosphate buffer saline solution and left to stand in 50 $\mu$L of Trypsin-EDTA (Sigma-Aldrich, Inc.) at 37°C for 10 minutes.

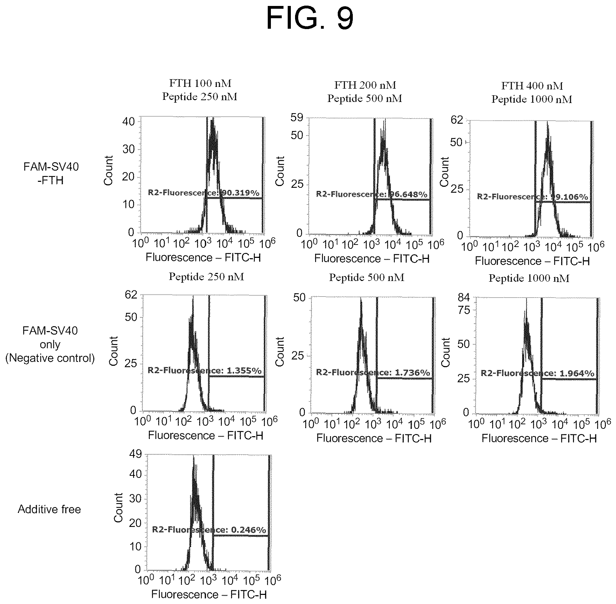

After 100 $\mu$L of the Opti-MEM™ medium was added, the cells were transferred to the plate for fluorescence-activated cell sorting (FACS), and then subjected to centrifugal separation at 400 $\times$ g for 5 minutes. The cells for each were suspended in the FACS buffer (Attune™ Focusing Fluid, Thermo Fisher Scientific Inc.), and analyzed by FACS (Attune NxT, Thermo Fisher Scientific Inc.). As a result, the fluorescence intensity was confirmed to increase depending on the concentration only from the FAM-SV40 encapsulating FTH, while fluorescence was not observed from the FAM-SV40 only (FIG. 9).

The above results reveals that the encapsulation in FTH enables the peptide that is conventionally impermeable to membranes to be transported into cells.

<Example 7: Confirmation of dependence of peptide transport amount on TfR expression level>

In order to confirm that an aimed peptide can be transported specifically to a transferrin receptor (TfR) presenting cell by the encapsulation in ferritin, TfR dependence of the FTH incorporation efficiency was assessed with use of cell strains with different levels of TfR expression.

In this assessment, an SKBR 3 cell with a high TfR expression level (expression level 449.7 TPM, see database "The Human Protein Atlas (https://www.proteinatlas.org/)") and an HEK 293E cell that is a substrain of a HEK 293E cell with a low TfR expression level (expression level 74.5 TPM, see database "The Human Protein Atlas") were used.

First, all RNAs were collected from SKBR 3 cells and HEK 293E cells with use of the PureLink (Registered trademark) RNA Mini kit (Thermo Fisher Scientific Inc.). With 2 µL of superscript™ VILO™ Master Mix (Thermo Fisher Scientific Inc.), 50 ng of all RNAs derived from each cell was mixed, and water was added thereto up to 10 µL as a total volume. After incubated at 25°C for 10 minutes, at 42°C for 60 minutes and 85°C for 5 minutes, the resulting solution was diluted with 115 µL of water to obtain 0.4 ng/µL of each cDNA solution. A TaqMan (Registered trademark) Fast Advanced Master Mix (Applied Biosystems Inc.), 4.8 µL of water and 25 µM primer mix were added to 5 µL of each cDNA solution to prepare a quantification PCR solution. A mixture (Forward: TGGCAGTTCAGAATGATGGA (SEQ ID NO: 26) and Reverse: AGGCTGAACCGGGTATATGA (SEQ ID NO: 27)) was used as a TfR gene primer mix. As an internal standard, 18S rRNA was quantified. A mixture (Forward: TGAGAAACGGCTACCACATC (SEQ ID NO: 28) and Reverse: TTACAGGGCCTCGAAAGAGT (SEQ ID NO: 29)) was used as a 18S rRNA gene primer mix. The mRNA expression level of TfR was determined by quantitative PCR (StepOnePlus™ System, Applied Biosystems, Inc.), revealing that the TfR expression level of the SKBR 3 cell was 3.2-fold higher than that of the HEK 293E cell.

Each cell (20,000 cell/well, 96-well plate) was added to a 100 µL medium that was produced by adding the FAM-SV40 encapsulating FTH to an evaluation medium (Opti-MEM™ (Thermo Fisher Scientific Inc.) + a 1% non-essential amino acid solution (Thermo Fisher Scientific Inc.) + 1% penicillin- streptomycin (Nacalai Tesque, Inc.)) at different final concentrations ranging from 0 to 800 nM (FAM-SV40 concentration 2 µM), and then cultured at 37°C. After the cell culture for 24 hours for each, the resulting solution was washed twice with 100 µL of a phosphate buffer saline solution and left to stand in 50 µL of Trypsin-EDTA (Sigma-Aldrich, Inc.) at 37°C for 10 minutes.

After 100 µL of the Opti-MEM™ medium was added, the cells were transferred to the plate for fluorescence-activated cell sorting (FACS), and then subjected to centrifugal separation at 400 × g for 5 minutes. The cells for each were suspended in the FACS buffer (Attune™ Focusing Fluid, Thermo Fisher Scientific Inc.), and analyzed by FACS (Attune NxT, Thermo Fisher Scientific Inc.).

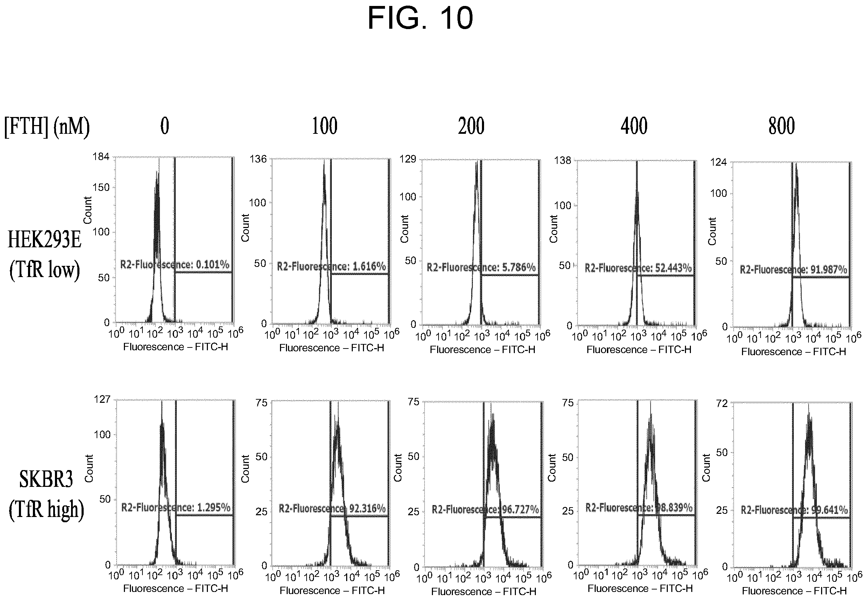

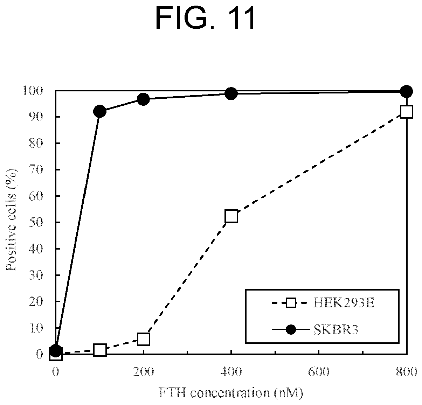

As a result, fluorescence was observed from 90% or more of SKBR 3 cells cultured in the medium that contains 100 nM of FAM-SV40 encapsulating FTH, indicating that almost all of the cells incorporated the FAM-SV40 encapsulating FTH. The ratio was 60 times as many as that of HEK 293E cells obtained by the same condition (FIG. 10). In the case of HEK 293E cells, the medium containing the FAM-SV40 encapsulating FTH at a high concentration of 800 nM or more was needed to emit fluorescence from 90% or more of the cells (FIG. 11). These results indicate that the SKBR 3 cell with a high TfR expression level incorporated ferritin into cells more efficiently.

That is, the amount of transporting the aimed peptide into cells was confirmed to be controllable by the encapsulation in ferritin depending on the TfR expression levels of target cells.

<Example 8: Search for peptide capable of being encapsulated into ferritin (1)>

Peptides capable of being encapsulated into ferritin by disassembly/reassembly process were searched. In the disassembly/reassembly process, 1 mL of a 50 mM glycine hydrochloride buffer (pH 2.3) containing FTH at a final concentration of 5 mg/mL and the peptide at a final concentration of 0.5 mM, was left to stand at room temperature for 15 minutes. Next, 310 µL of a 1 M Tris hydrochloride buffer (pH 9.0) was added for neutralization, and left to stand at room temperature for 3 hours. After the standing, 1.2 mL of a 50 mM Tris hydrochloride buffer (pH 8.0) was added, and centrifuged (at 15,000 rpm for one minute). Thereafter, 2.5 mL of its supernatant was injected into a desalting column PD-10 (Sephadex G-25 filled product, GE Healthcare Inc.) equilibrated with a 50 mM Tris hydrochloride buffer (pH 8) for the elusion with 3.0 mL of a 50 mM Tris hydrochloride buffer (pH 8.0) to separate non-encapsulated peptides and ferritin. A whole amount (3.0 mL) of the resulting solution was concentrated to 0.1 mL by centrifugal ultrafiltration using Vivaspin 20-100K (GE Healthcare Inc.). The operation of adding 10 mL of a 50 mM Tris hydrochloride buffer to the concentrated solution and then concentrating was repeated twice to remove peptides that were not removed by PD-10 to obtain 0.1 mL of a sample solution. For the LC-MS analysis, 50 µL of a 1 M glycine hydrochloride buffer (pH 2.3) was added to 50 µL of the sample solution and then the resulting solution was left to stand at room temperature for 15 minutes. To the solution, 900 µL of ethanol was added. Next, the resulting solution was stirred vigorously and then centrifuged (at 15,000 rpm for five minutes) for collection of the supernatant. The supernatant was analyzed using LCMS-2020 (Shimadzu Corporation) as LC-MS, Inertsil (Registered trademark) as a column, ODS-3, particle diameter of 2 µm and 2.1 mm × 50 mm (GL science). Thereafter, 10 µL of the sample injected into the column was eluted at a flow rate of 0.2 mL/min for 10 minutes such that a mixing ratio of a solution A (0.1% formic acid / 99.9% acetonitrile) and a solution B (50% water / 50% acetonitrile) is from 95:5 to 95:5.

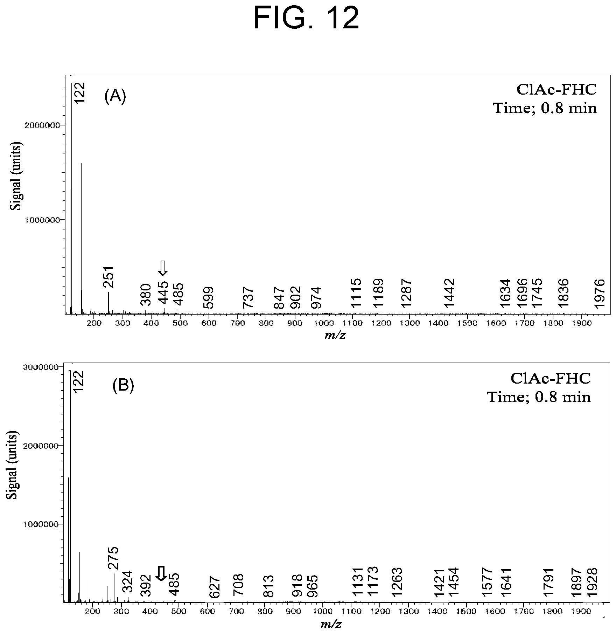

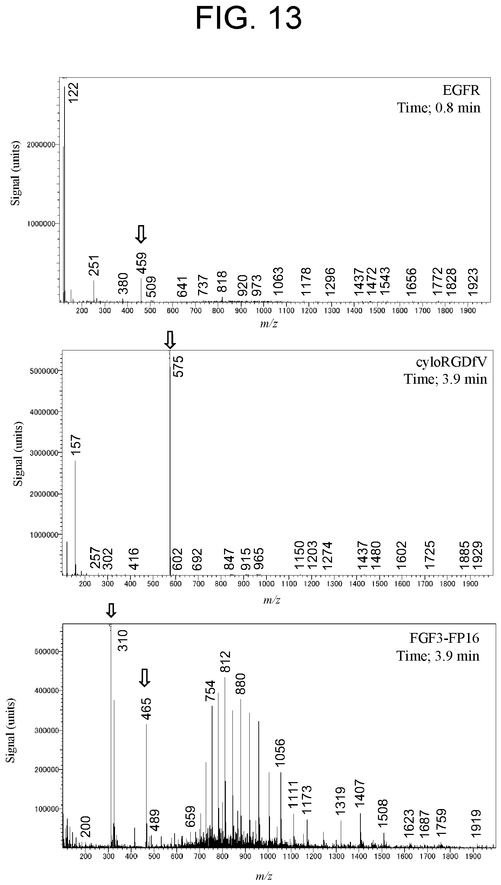

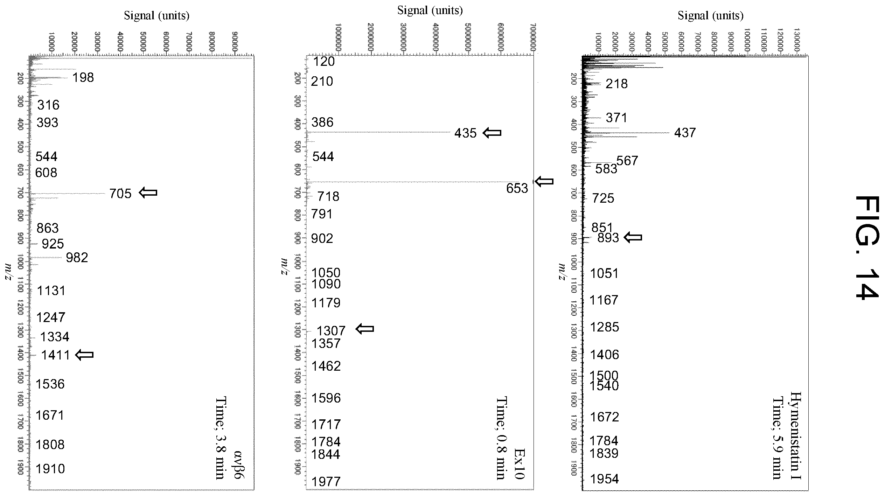

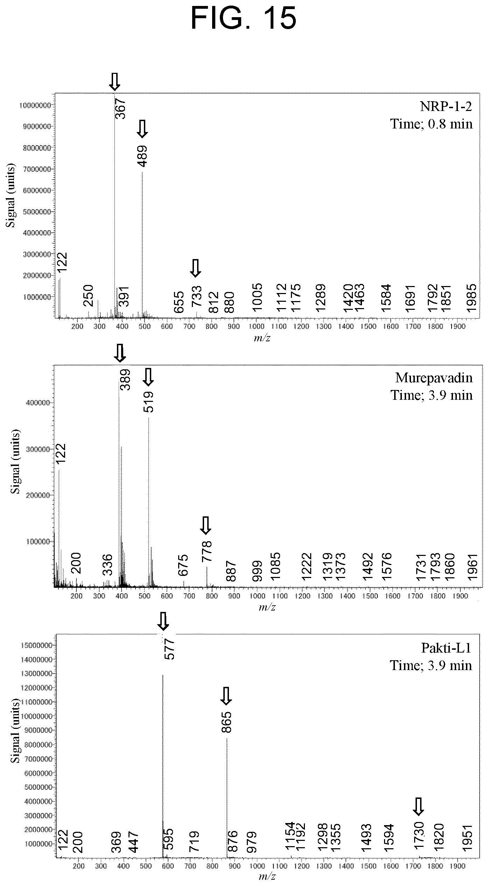

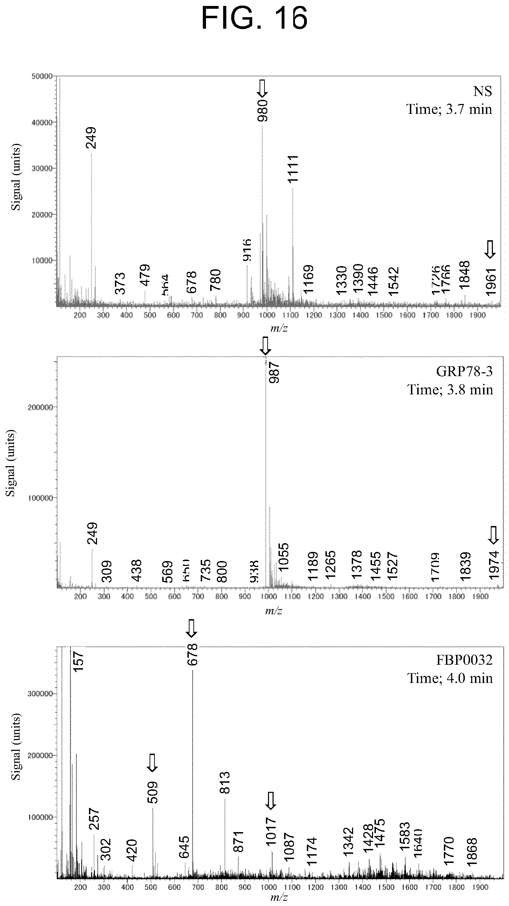

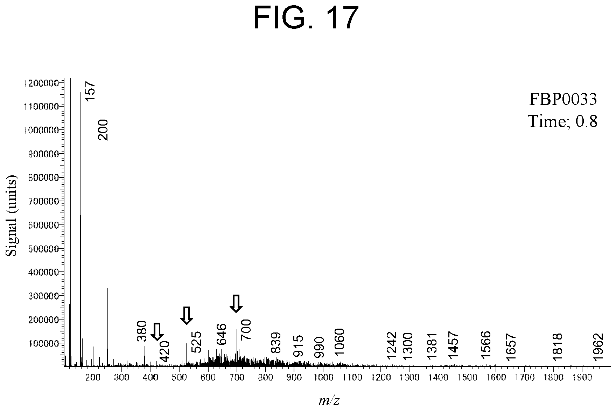

Table 1 summarizes peptides used in this study of encapsulation. The encapsulation of the peptide into ferritin was studied by comparing in terms of the amount of the peptide detected by LC/MS between the sample prepared by encapsulating the peptide into ferritin at the disassembly/reassembly process and the sample prepared simply by mixing ferritin with the peptide. For example, ClAc-FHC was eluted at a retention time of 0.8 minute. A peak derived from ClAc-FHC (chemical formula weight 445) obtained by the disassembly/reassembly process was observed at m/z = 445 (FIG. 12A). However, the peak was not observed for the sample prepared by simply mixing the peptide with ferritin without performing the disassembly/reassembly process (FIG. 12B). FIG. 13 to FIG. 17 illustrate LC-MS results for FTH in which the peptide was encapsulated at the disassembly/reassembly process.

Table 1. Structural information of peptides used in investigation of encapsulation into ferritin

| Name | Amino acid sequence (SEQ ID NO | Linear Cyclic | Source [3] |
|---|---|---|---|
| PKC-F | 5(6)-FAM-RFARKGALRQKNVHEVKN (SEQ ID NO:9) | Linear | A |
| FAM-SV40 | 5(6)-FAM-CGGPKKKRKVG (SEQ ID NO:10) | Linear | A |
| FAM-Np | 5(6)-FAM-CGGKRPAAIK KAGQAKKKKG (SEQ ID NO:11) | Linear | A |
| IE | IE | Linear | A |
| ClAc-FHC | Ac-FHC-NH$_2$ (cyclic S) | Cyclic | A |
| EGFR | HTSD (SEQ ID NO:12) | Linear | A |
| cyloRGDfV | cyclo(RGDFV) (SEQ ID NO:13) | Cyclic | B |
| FGF3-FP16 | VLWLKNR ( EQ ID NO:14) | Linear | A |
| Hymenistatin I | cyclo(PPYVPLII) (SEQ ID NO:15) | Cyclic | B |
| Ex10 | EEEEEEEEEE ( EQ ID NO:16) | Linear | A |
| Rx10 | RRRRRRRRRR ( EQ ID NO:17) | Linear | A |
| αvβ6 | SPRGDLAVLGHKY (SEQ ID NO:18) | Linear | A |
| NRP-1-2 | GGKRPARGGKRPAR ( EQ ID NO:19) | Linear | A |
| Murepavadin 5 | cyclo(AS(D-Pro)PTWI(Dab)(Orn)(D-Dab) (Dab) W (Dab) (Dab)) | Cyclic | A |
| Pakti-L1 | Ac-YILVRNRLLRVDCG-NH$_2$ (SEQ ID NO:20) (cyclic S) | Cyclic | A |
| NS | QMARIPKRLARHQMAR (SEQ ID NO:21) | Linear | A |
| GRP78-3 | GIRLRGGIRLRGGIRLRG (SEQ ID NO:22) | Linear | A |
| FBP0032 | XGQKGCKKTPKLASLSCTGF-NH$_2$ (SEQ ID NO:23) (S-S) | Cyclic | C |
| FBP0033 | XGRPGCGPRKPRTPKKCGSH-NH$_2$ (SEQ ID NO:24) (S-S) | Cyclic | C |

[1] X in FBP0032 and FBP0033 represents 4-pentynoic acid.

[2] the sequence ID number is not described for IE, ClAc-FHC and Murepavadin since description of sequence ID number is not needed for any amino acid sequence with the number of amino acid residues of 3 or less and any amino acid sequence including D-amino acids.

[3] Sources are as follows:
A: Commissioning synthesis by Eurofins Genomics K.K.
B: Purchased from Sigma-Aldrich Co. LLC.
C: Solid-phase synthesis using Syro I (Biotage).

[4] Cyclic structures are formed by cyclization of a main chain of a peptide (that is, amido bond between an amino group at N-terminus and a carboxy group at C-terminus) (cyloRGDfV, Hymenistatin I, and Murepavadin), by bonding between a sulfur atom (S) in a side chain of a cysteine residue and a carbon atom in an acetyl group at N-terminus (Pakti-L1), or by disulfide bonding between two sulfur atoms (S) in side chains of two cysteine residues (FBP0032 and FBP0033).

[5] "D-" represents D form. Dab represents 2,4-Diaminobutanoic acid. Orn represents ornithine.

Table 2 lists capability/incapability of the encapsulation of peptides into ferritin assessed in the Examples.

<Example 9: Study of physical property relevant to capability of the encapsulation into ferritin>

The correlation between each physical property value described below and the capability/incapability of encapsulation of the peptide into the ferritin was studied for peptides encapsulated in ferritin and peptides that could not be encapsulated in ferritin in the Examples.

(1) Length of peptide (Chain length)
(2) Chemical formula weight of peptide
(3) Hydrophobicity
(4) GRAVY
(5) Average of hydrophilicity
(6) Ratio of hydrophilic residues / total number of residues in the peptide
(7) Charge of peptide at pH 9

The physical property values of the above (1) to (7) were determined according to the following methods.
(1) Length of peptide (Chain length)
The length of the peptide was determined based on the total number of amino acid residues constituting the peptide.
(2) Chemical formula weight of peptide
It was determined as the total sum of products of atomic weight and the number of atoms based on the composition formula of the peptide.
(3) Hydrophobicity
The Hydrophobicity of the peptide was calculated based on SSRCalc Hydrophobicity (O. V. Krokhin Anal. Chem. (2006), 78(22) 7785-7795). In the Example, it was calculated using database Prot pi (https://www.protpi.ch/Calculator/PeptideTool) with settings for a 300 angstrom C18 column and a 0.1% TFA elusion condition.
(4) GRAVY
The GRAVY (grand average of hydropathy) was calculated by adding a hydropathy score for each amino acid residue and then dividing by a length of sequence (J, Kyte and R. F. Doolittle, J Mol Biol. 1982 157(1) 105-32.).

Table 2. Examples of hydropathy score for each amino acid residue

| Amino Acid | One Letter Code | Hydropathy Score |
|---|---|---|
| Isoleucine | I | 4.5 |
| Valine | V | 4.2 |
| Leucine | L | 3.8 |
| Phenylalanine | F | 2.8 |
| Cysteine | C | 2.5 |
| Methionine | M | 1.9 |
| Alanine | A | 1.8 |
| Glycine | G | -0.4 |
| Threonine | T | -0.7 |
| Serine | S | -0.8 |
| Tryptophan | W | -0.9 |
| Tyrosine | Y | -1.3 |
| Proline | P | -1.6 |
| Histidine | H | -3.2 |
| Glutamic acid | E | -3.5 |
| Glutamine | Q | -3.5 |
| Aspartic acid | D | -3.5 |

(continued)

| Amino Acid | One Letter Code | Hydropathy Score |
|---|---|---|
| Asparagine | N | -3.5 |
| Lysine | K | -3.9 |
| Arginine | R | -4.5 |

(5) Average of hydrophilicity

The Average of hydrophilicity was calculated by adding the hydrophilicity score of each amino acid residue and then dividing by the length of sequence (Hopp and Woods Proc Natl Acad Sci USA. 1981 78 (6) 3824-8.).

Table 3. Examples of hydrophilicity of each amino acid residue

| Amino Acid | 3 Letter Code | Hydrophilicity Score |
|---|---|---|
| Arginine | Arg | 3 |
| Aspartic acid | Asp | 3 |
| Glutamic acid | Glu | 3 |
| Lysine | Lys | 3 |
| Serine | Ser | 0.3 |
| Asparagine | Asn | 0.2 |
| Glutamine | Gln | 0.2 |
| Glycine | Gly | 0 |
| Proline | Pro | 0 |
| Threonine | Thr | -0.4 |
| Alanine | Ala | -0.5 |
| Histidine | His | -0.5 |
| Cysteine | Cys | -1 |
| Methionine | Met | -1.3 |
| Valine | Val | -1.5 |
| Isoleucine | Ile | -1.8 |
| Leucine | Leu | -1.8 |
| Tyrosine | Tyr | -2.3 |
| Phenylalanine | Phe | -2.5 |
| Tryptophan | Trp | -3.4 |

(6) Ratio of hydrophilic residues / total number of residues in the peptide

The Ratio of hydrophilic residues / total number of residues in the peptide was determined by calculating the ratio of hydrophilic amino acids (H, C, T, S, K, Q, E, D, N and R) among all amino acids constituting the peptide.

(7) Charge of peptide at pH 9

The charge of peptide at pH 9 was calculated by the following formula under pH 9 using the dissociation constant (pKa) and the positive/negative charges of each functional group, for charges of all functional groups that include side chains and modified groups of all amino acid residues constituting the peptide as well as amino group or its protecting group at the N-terminus and carboxy group or its protecting group at the C-terminus.

$$C = \sum_i \left( N_i \frac{10^{pKai-pH}}{1+10^{pKai-pH}} \right) - \sum_j \left( N_j \frac{10^{-(pKaj-pH)}}{1+10^{-(pKaj-pH)}} \right)$$

C: Charge of target peptide under a certain setting of pH pH: A certain setting of pH
$N_i$: The number of side chains i with positive charges contained in the target peptide
$pKa_i$: pKa of the side chain i with positive charges contained in the target peptide
$N_j$: The number of side chains j with negative charges contained in the target peptide
$pKa_j$: pKa of the side chain j with negative charges contained in the target peptide
pKa of each amino acid was evaluated by using ChemAxon (https://chemaxon.com/) (O. Toure et al., Oil Gas Sci. Technol. 2013, 68, 281-297.).

Table 4. Examples of pKa values of amino acid residues

| Amino Acid | 3 Letter Code | α-COOH | α-NH2 | Side chain* |
|---|---|---|---|---|
| Alanine | Ala | 2.5 | 9.5 | - |
| Arginine | Arg | 2.4 | 9.1 | 12.4 |
| Asparagine | Asn | 2.0 | 8.4 | - |
| Aspartic acid | Asp | 1.7 | 9.6 | 5.1 |
| Cysteine | Cys | 2.4 | 9.1 | 10.2 |
| Glutamic acid | Glu | 1.9 | 9.5 | 4.3 |
| Glutamine | Gln | 2.2 | 9.3 | - |
| Glycine | Gly | 2.3 | 9.2 | - |
| Histidine | His | 1.9 | 9.4 | 6.6 |
| Isoleucine | Ile | 2.8 | 9.6 | - |
| Leucine | Leu | 2.8 | 9.5 | - |
| Lysine | Lys | 2.7 | 9.4 | 10.3 |
| Methionine | Met | 2.5 | 9.5 | - |
| Phenylalanine | Phe | 2.5 | 9.5 | - |
| Proline | Pro | 1.9 | 11.3 | - |
| Serine | Ser | 2.0 | 8.9 | - |
| Threonine | Thr | 2.2 | 9.0 | - |
| Tryptophan | Trp | 2.5 | 9.4 | |
| Tyrosine | Tyr | 2.0 | 9.2 | 9.8 |
| Valine | Val | 2.7 | 9.6 | - |
| Ornithine | Orn | 2.7 | 9.4 | 10.3 |
| 2,3,-Diaminobutyric acid | Dab | 2.6 | 8.0 | 10.0 |
| * No dissociation group is referred to as "-" | | | | |

The relationship between each physical property value of the peptide used in the study of the encapsulation into ferritin and the capability/incapability of the encapsulation into ferritin is as follows.

Table 5. Physical property values of peptides used in study of encapsulation into ferritin

| Name | (1) | (2) | (3) | (4) | (5) | (6) | (7) | encapsulation |
|---|---|---|---|---|---|---|---|---|
| IE | 2 | 260 | 1.2 | 0.5 | 0.6 | 50 | -1.2 | C |

17

(continued)

| Name | (1) | (2) | (3) | (4) | (5) | (6) | (7) | encapsulation |
|---|---|---|---|---|---|---|---|---|
| ClAc-FHC | 3 | 445 | - | - | - | - | 0.0 | A |
| EGFR | 4 | 458 | 1. 04 | -2.05 | 0.6 | 50 | -1.3 | A |
| cyloRGDfV | 5 | 575 | - | - | - | - | 0.0 | B |
| FGF3-FP16 | 7 | 928 | 26.1 7 | -0.14 | -0.3 | 43 | 1.8 | B |
| Hymenistatin I | 8 | 893 | - | - | - | - | -0.1 | B |
| Ex10 | 10 | 1309 | 6.62 | -3.5 | 3 | 100 | -10.2 | A |
| Rx10 | 10 | 1580 | -4.26 | -4.5 | 3 | 100 | 9.6 | C |
| FAM-SV40 | 11 | 1516 | - | - | - | - | 3.7 | A |
| $\alpha\nu\beta6$ | 13 | 1413 | 23.89 | -0.46 | 0.1 | 31 | 0.3 | B |
| NRP-1-2 | 14 | 1464 | -0.41 | -1.93 | 1.2 | 43 | 5.5 | A |
| Murepavadin | 14 | 1554 | - | - | - | - | 5.4 | A |
| Pakti-L1 | 14 | 1729 | - | - | - | - | 1.9 | B |
| NS | 16 | 1963 | 21.34 | -1.01 | 0.4 | 44 | 4.6 | A |
| PKC-F | 18 | 2524 | - | - | - | - | 3.9 | A |
| GRP78-3 | 18 | 1976 | 31.43 | -0.25 | 0.4 | 33 | 5.6 | B |
| FBP0032 | 19 | 2031 | - | - | - | - | 3.8 | A |
| FBP0033 | 19 | 2096 | - | - | - | - | 5.9 | A |
| FAM-Np | 20 | 2384 | - | - | - | - | 6.6 | C |
| Physical property values (1) to (7) are provided above. There are three trials. "A" represents peptides with three times of confirmation of encapsulation. "B" represents peptides with two times of confirmation of encapsulation or less. "C" represents peptides without confirmation of encapsulation. | | | | | | | | |

As a result, the correlation was found between the relational expression of the electric charge at pH 9.0 with the chemical formula weight and easiness of the encapsulation of peptide (FIG. 18).

That is, the peptide satisfying conditions of a) $-10.2 \leq$ charge of peptide at pH 9 (X) $\leq 5.9$ and $445 \leq$ chemical formula weight (Y) $\leq 2,524$ was successfully encapsulated in the ferritin (FIG. 18).

Among the peptides satisfying the above conditions, the peptide satisfying conditions of b) $-10.2 \leq X \leq 0.0$ and $445 \leq Y \leq 2,524$, or c) $3.7 \leq X \leq 5.9$ and $445 \leq Y \leq 2,524$ was successfully reproducibly encapsulated in ferritin (FIG. 18).

<Example 10: Preparation of ferritin (2)>

Total synthesis was carried out for a DNA encoding a human-derived ferritin L chain (FTL (SEQ ID NO: 2)). PCR was carried out using the synthesized DNA as a template as well as the following primers: 5'-GAAGGAGATATACATATGAGCTCCCAGATTCGTCAG-3' (SEQ ID NO: 7) and 5'-CTCGAATTCGGATCCTTAGTCGTGCTTGAGAGTGAG-3' (SEQ ID NO: 8). PCR was carried out using pET20 (Merck KGaA) as a template as well as the following primers: 5'-TTTCATATGTATATCTCCTTCTTAAAGTTAAAC-3' (SEQ ID NO: 5) and 5'-TTTGGATCCGAATTCGAGCTCCGTCG-3' (SEQ ID NO: 6). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTL) carrying a gene encoding FTL.

Subsequently, Escherichia coli BL21 (DE3) into which the constructed pET20-FTL was introduced, was cultured in 100 mL of an LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with a 50 mM Tris-HCl buffer (pH 8.0). Then, the aimed protein was separated and purified by applying a concentration gradient of the salt from 0 mM to 500 mM NaCl in 50 mM Tris-HCl buffer (pH 8.0). The solvent of the solution containing the protein was replaced with a 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with a 10 mM Tris-HCl buffer (pH 8.0) to separate and purify FTL by size. The solution containing FTH was concentrated by centrifugal ultrafiltration using Vivaspin 20-100K (GE Healthcare Inc.), and then the concentration of the contained protein was determined using a protein assay CBB solution (Nacalai Tesque, Inc.) in terms of bovine albumin as a standard. As a result, a 1 mL solution containing 3 mg/mL of FTL was obtained from 100 mL of the culture solution.

<Example 11: Study of encapsulation of peptide modified with fluorescent dye>

FTL, in which a peptide (5(6)-FAM-RFARKGALRQKNVHEVKN (SEQ ID NO: 9), PKC-F, chemical formula weight 2,524) modified with fluorescent dye fluoroserin (FAM) was encapsulated, was constructed.

0.5 mL of a 50 mM glycine hydrochloride buffer (pH 2.3) that contains FTL (chemical formula weight 480,466) at a final concentration of 5 mg/mL and the peptide at a final concentration of 0.5 mM, was left to stand at room temperature for 15 minutes. Next, 50 μL of a 1 M Tris hydrochloride buffer (pH 9.0) was added for neutralization, and the resulting solution was left to stand at room temperature for 3 hours. After the standing, the resulting solution was centrifuged (at 15,000 rpm for one minute). Then, the supernatant was collected and diluted with a 10 mM Tris hydrochloride buffer (pH 8) up to 3 mL in volume. The resulting solution was injected into the HiPrep 26/60 Sephacryl S-300 HR column (GE Healthcare Inc.) equilibrated with D-PBS (-) (Fujifilm Wako Pure Chemical Corporation) to separate and purify 24-mer FTL by size at a flow rate of 1.3 mL/minute. The purified ferritin was analyzed using a HiPrep 16/60 Sephacryl S-300 HR column (GE Healthcare Inc.) equilibrated with PBS at a flow rate of 0.5 mL/minute. The absorbance at 480 nm, unobservable from FTL only, is confirmed at the same position as that of a peak of FTL, revealing that the peptide was encapsulated in FTL (FIG. 19). The above results demonstrate that the peptide can be encapsulated also in FTL.

**Claims**

1. A peptide-encapsulating ferritin comprising

   a peptide that is composed of 3 to 19 amino acid residues and satisfies the following requirement:

   a) $-10.2 \leq X \leq 5.9$ and $445 \leq Y \leq 2,524$

   wherein X represents a charge of the peptide at pH 9, and Y represents a chemical formula weight of the peptide.

2. The peptide-encapsulating ferritin according to claim 1, wherein the peptide is composed of 4 to 16 amino acid residues.

3. The peptide-encapsulating ferritin according to claim 1 or 2, wherein the peptide satisfies the following requirement:

   b) $-10.2 \leq X \leq 0.0$ and $445 \leq Y \leq 2,524$; or
   c) $3.7 \leq X \leq 5.9$ and $445 \leq Y \leq 2,524$ wherein X and Y represent the same as those defined in claim 1.

4. An agent for intracellularly transporting a peptide, the agent comprising
   a peptide-encapsulating ferritin, wherein the peptide is composed of 3 to 19 amino acid residues and satisfies the following requirement:

   a) $-10.2 \leq X \leq 5.9$ and $445 \leq Y \leq 2,524$ wherein X represents a charge of the peptide at pH 9, and Y represents a chemical formula weight of the peptide.

5. The agent according to claim 4, wherein the peptide is transported into a human cell.

6. The agent according to claim 4 or 5, wherein the peptide is transported into a cancer cell.

7. A method for producing a peptide-encapsulating ferritin, the method comprising:

   1) standing a ferritin in a buffer with a pH of 3.0 or less to dissociate the ferritin in the presence or absence of a peptide; and
   2) allowing the dissociated ferritin and the peptide to coexist in a buffer with a pH of 5.0 or more and 10.0 or less to generate the peptide-encapsulating ferritin, wherein
   the peptide is composed of 3 to 19 amino acid residues and satisfies the following requirement:

   a) $-10.2 \leq X \leq 5.9$ and $445 \leq Y \leq 2,524$

   wherein X represents a charge of the peptide at pH 9, and Y represents a chemical formula weight of the peptide.

# FIG. 1

# FIG. 2

# FIG. 3

# FIG. 4

# FIG. 5

# FIG. 6

# FIG. 7

PKC-F-FTH                                          PKC-F-only (Negative control)

FTH 100 nM, Peptide 0.86 μM                            Peptide 0.86 uM

FTH 200 nM, Peptide 1.72 μM                            Peptide 1.72 μM

FTH 400 nM, Peptide 3.44 μM                            Peptide 3.44 μM

F: Fluorescence of vision, B: Bright field of vision

# FIG. 8

# FIG. 9

FIG. 10

EP 4 056 233 A1

FIG. 11

# FIG. 12

# FIG. 13

# FIG. 14

# FIG. 15

# FIG. 16

# FIG. 17

# FIG. 18

# FIG. 19

**INTERNATIONAL SEARCH REPORT**

| International application No. |
| --- |
| PCT/JP2020/033658 |

**A. CLASSIFICATION OF SUBJECT MATTER**
A61P 43/00(2006.01)i; C07K 1/02(2006.01)i; C07K 7/04(2006.01)i; A61K
47/42(2017.01)i; C12N 15/12(2006.01)i; A61K 38/06(2006.01)i; A61K
38/07(2006.01)i; A61K 38/08(2019.01)i; A61K 38/10(2006.01)i
FI:      C12N15/12; A61K38/06; A61K38/07; A61K38/08; A61K38/10; A61P43/00
         111; A61K47/42; C07K1/02; C07K7/04 ZNA
According to International Patent Classification (IPC) or to both national classification and IPC

**B. FIELDS SEARCHED**

Minimum documentation searched (classification system followed by classification symbols)
A61P43/00; C07K1/02; C07K7/04; A61K47/42; C12N15/12; A61K38/06;
A61K38/07; A61K38/08; A61K38/10

Documentation searched other than minimum documentation to the extent that such documents are included in the fields searched
|   |   |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/REGISTRY (STN);
CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | CN 106110333 A (INSTITUTE OF PROCESS ENGINEERING, CHINESE ACADEMY OF SCIENCES) 16 November 2016 (2016-11-16) claims | 1-7 |
| A | WO 2019/163871 A1 (AJINOMOTO CO., INC.) 29 August 2019 (2019-08-29) paragraph [0009] | 1-7 |
| A | KANG, Young-Ji, et al., "Incorporation of Thrombin Cleavage Peptide into a Protein Cage for Constructing a Protease-Responsive Multifunctional Delivery Nanoplatform", Biomacromolecules, 2012, vol. 13, pp. 4057-4064 abstract, fig. 1 | 1-7 |
| P, A | WO 2020/090708 A1 (AJINOMOTO CO., INC.) 07 May 2020 (2020-05-07) paragraphs [0009], [0030]-[0034] | 1-7 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 21 October 2020 (21.10.2020) | 02 November 2020 (02.11.2020) |

| Name and mailing address of the ISA/ Japan Patent Office 3-4-3, Kasumigaseki, Chiyoda-ku, Tokyo 100-8915, Japan | Authorized officer Telephone No. |
| --- | --- |

Form PCT/ISA/210 (second sheet) (January 2015)

## INTERNATIONAL SEARCH REPORT
Information on patent family members

International application No.

PCT/JP2020/033658

| Patent Documents referred in the Report | Publication Date | Patent Family | Publication Date |
|---|---|---|---|
| CN 106110333 A | 16 Nov. 2016 | (Family: none) | |
| WO 2019/163871 A1 | 29 Aug. 2019 | (Family: none) | |
| WO 2020/090708 A1 | 07 May 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4515736 A
- US 20160060307

- WO 2006126595 A

### Non-patent literature cited in the description

- **Y. HAYASHI et al.** *ACS Chem. Biol.,* 2012, vol. 7 (3), 607-613
- **Z. GUO et al.** *Biomed Rep,* 2016, vol. 4 (5), 528-534
- **L. LI et al.** *Proc Natl Acad Sci USA.,* 2010, vol. 107 (8), 3505-10
- **JAE OG JEON et al.** *ACS Nano,* 2013, vol. 7 (9), 7462-7471
- **SOOJI KIM et al.** *Biomacromolecules,* 2016, vol. 17 (3), 1150-1159
- **YOUNG JI KANG et al.** *Biomacromolecules,* 2012, vol. 13 (12), 4057-4064
- *Int J Mol Sci,* 2011, vol. 12 (8), 5406-5421
- **Y. J. KANG.** *Biomacromolecules,* 2012, vol. 13 (12), 4057
- **F. DANHIER et al.** *Mol. Pharmaceutics,* 2012, vol. 9 (11), 2961
- **C-H. WU et al.** *Sci. Transl. Med.,* 2015, vol. 7 (290), 290-91
- **L. VANNUCCI et al.** *Int. J. Nanomedicine,* vol. 7, 1489
- **J. CUTRERA et al.** *Mol. Ther.,* 2011, vol. 19 (8), 1468
- **R. LIU et al.** *Adv. Drug Deliv. Rev.,* 2017, vol. 110-111, 13
- **R. TAN et al.** *Proc. Natl. Acad. Sci. USA,* 1995, vol. 92, 5282
- **R. TAN et al.** *Cell,* 1993, vol. 73, 1031
- **R. TALANIAN et al.** *Biochemistry,* 1992, vol. 31, 6871
- **K. OLDENBURG et al.** *Proc. Natl. Acad. Sci. USA,* 1992, vol. 89 (12), 5393-5397
- **K. YAMAMOTO et al.** *Biochem.,* vol. 111, 436
- **A. BAIMIEV et al.** *Mol. Biol. (Moscow),* 2005, vol. 39 (1), 90
- **O. KRUSE et al.** *B Z. Naturforsch.,* 1995, vol. 50c, 380

- **O. SILVA et al.** *Sci. Rep.,* 2016, vol. 6, 27128
- **A. FILOTEO et al.** *J. Biol. Chem.,* vol. 267 (17), 11800
- **MCILWAIN1 et al.** *Cold Spring Harb Perspect Biol.,* 2013, vol. 5, a008656
- **V. STOKA et al.** *IUBMB Life,* 2005, vol. 57 (4-5), 347
- **G. LEE et al.** *Eur J Pharm Biopharm.,* 2007, vol. 67 (3), 646
- **R. JENNY et al.** *Protein Expr. Purif.,* 2003, vol. 31, 1
- **H. XU et al.** *J. Virol.,* 2010, vol. 84 (2), 1076
- **C. BYRD et al.** *Drug Dev. Res.,* 2006, vol. 67, 501
- **E. LEE et al.** *Adv. Funct. Mater.,* 2015, vol. 25, 1279
- **Y. KANG et al.** *Biomacromolecules,* 2012, vol. 13 (12), 4057
- **R. TALANIAN et al.** *J. Biol. Chem.,* 1997, vol. 272, 9677
- **JENNY et al.** *Protein Expr. Purif.,* 2003, vol. 31, 1
- **X. MENG et al.** *Nanoscale,* 2011, vol. 3 (3), 977
- **E. FALVO et al.** *Biomacromolecules,* 2016, vol. 17 (2), 514
- **Z. GUO et al.** *Biomed. Rep.,* 2016, vol. 4 (5), 528
- **O. V. KROKHIN.** *Anal. Chem.,* 2006, vol. 78 (22), 7785-7795
- **J, KYTE ; R. F. DOOLITTLE.** *J Mol Biol.,* 1982, vol. 157 (1), 105-32
- **HOPP ; WOODS.** *Proc Natl Acad Sci USA.,* 1981, vol. 78 (6), 3824-8
- **O. TOURE et al.** *Oil Gas Sci. Technol.,* 2013, vol. 68, 281-297